# EUROPÄISCHE PATENTANMELDUNG

(11) **EP 1 364 965 A1**
(43) Veröffentlichungstag der Anmeldung: **26.11.2003**
(21) Anmeldenummer: 03090148.2
(22) Anmeldetag: 20.05.2003
(51) Int. Cl.: C07K 14/475, C07K 16/18, C12N 15/09, G01N 33/48

(54) **Slit1 und MEGF4 Isoformen und deren Verwendung**

(30) Priorität: 21.05.2002 DE 10223246
(71) Anmelder: metaGen Pharmaceuticals GmbH, 13347 Berlin (DE)
(72) Erfinder: Mennerich, Detlev, 13347 Berlin (DE)
(74) Vertreter: Jungblut, Bernhard Jakob, Dr.

(57) **Zusammenfassung**

Die Erfindung betrifft neue Slitl und MEGF4 Sequenzen sowie deren Verwendungen im Zusammenhang mit der Diagnose und/oder Behandlung von Krebs oder dem Screenen nach nach an Slit1und/oder MEGF4 bindenden Substanzen, insbesondere prospektiven Inhibitoren.

## Beschreibung

### Gebiet der Erfindung

Die Erfindung betrifft neue Slitl und MEGF4 Isoformen, die Verwendung von aus Slit1 und MEGF4 abgeleiteten Sequenzen zum Screenen nach daran bindenden Substanzen, sowie die Verwendung von an Slit1 und/oder MEGF4 bindenden Substanzen zur Diagnose und/oder Behandlung von Krebserkrankungen.

### Hintergrund der Erfindung und Stand der Technik

Sekretierte Slit1 und MEGF4 Proteine, welche verschiedene transkriptionelle Isoformen sind, sind gut untersucht in neurologischen Zusammenhängen. In der Embryogenese von Drosophila spielt das Slit Gen eine wesentlich Rolle in der Bildung des zentralen Nervensystems.

Aus der Literaturstelle Itoh et al., Molec. Brain Res., 62:175-186 (1998), ist bekannt, daß 3 humane Homologe, Slit1, Slit2 und Slit3, zu dem Slit Gen der Drosophila existieren. Jedes dieser Gene codiert ein Protein, welches konservierte Protein-Protein-Wechselwirkungsdomänen, beispielsweise Leucin-reiche Repeats (LRR1 - LRR4) und EGFartige Motive (EGF1 - EGF9), ähnlich dem Drosophila Gen enthält. Humanes Slit1 ist bekannt unter XM_005958 bzw. NM_003061. Analysen haben ergeben, daß ein größeres 8,4 kb und ein kleineres 5,9 kb Transcript von Slit1 im wesentlichen in dem Gehirn exprimiert wird. Slit2 und Slit3 werden im wesentlichen im Rückenmark und der Schilddrüse exprimiert.

Gemäß der Literaturstelle M. Nakajama et al., Genomics, 51:27-34 (1998), befindet sich das Slit1 Gen, dort MEGF4 genannt, auf Chromosom 10q23.3-q24.

Verschiedene Literaturstellen beschäftigen sich mit der Rolle von Slit bzw. MEGF in der Ausbildung von und Funktion in neuralen Geweben von Säugetieren. Hierzu wird lediglich beispielhaft auf W. Wu et al., Nature 400:331-336 (1999), W. Yuan et al., Developmental Biology 212:290-306 (1999), T. Kidd et al., Cell 96:785-794 (1999), Y. Liang et al., JBC 274:17885-17892 (1999), und E. Stein et al., Science, 291: (2001) verwiesen.

In der Literaturstelle JP11164690 ist in allgemeinster Form die Diagnose und die Behandlung von Krebserkrankungen, ohne nähere Spezifizierung der Art der Krebserkrankung, mittels einer biologisch aktiven Form des Slit1 Proteins beschrieben.

Krebs, beispielsweise Prostatakrebs oder Brustkrebs, aber auch andere Krebserkrankungen, ist eine mit zunehmendem Alter mit beachtlicher Incidenz auftretende Erkrankung. Bislang wird Krebs im wesentlichen pathologisch diagnostiziert und meist durch Entfernung des betroffenen Organes oder eines Teils des betroffenen Organes behandelt. Die Entfernung eines Organes hat verschiedene nachteilige Effekte auf einen Patienten. Eine verbesserte Diagnose und Behandlung von Krebs, insbesondere ohne das Erfordernis einer Entfernung eines Organes oder eines Teiles davon, ist daher in hohem Maße wünschenswert.

### Technisches Problem der Erfindung

Der Erfindung liegt das technische Problem zugrunde, pharmazeutische Zusammensetzungen zur Diagnose und/oder zur Behandlung von Krebserkrankungen anzugeben.

Grundzüge der Erfindung sowie bevorzugte Ausführungsbeispiele.

Zur Lösung dieses technischen Problems lehrt die Erfindung zunächst eine Nukleinsäure codierend für eine Slit1 oder MEGF4 Isoform enthaltend eine Nukleinsäuresequenz gemäß Seq.-ID 2, 3, 4, 5, 25, oder 26 sowie ein Slit1 oder MEGF4 Peptid oder Protein enthaltend eine Aminosäurensequenz gemäß Seq.-ID 7, 8, 9, 10, 11, 12, 13, 14, 27, oder 28. Die Nukleinsäure bzw. das Peptid oder Protein können insbesondere aus diesen Sequenzen bestehen. Bei den besagten Sequenzen handelt es sich um neue Isoformen des humanen Slit1 bzw. MEGF4. Erfindungsgemäße Nukleinsäuren oder Proteine bzw. Peptide lassen sich mit den üblichen molekularbiologischen Methoden herstellen.

Die Erfindung betrifft weiterhin verschiedene Verwendungen der neuen Nukleinsäuren bzw. Peptide oder Proteine, ebenso wie (gleiche) Verwendungen bereits bekannter Slit1 bzw. MEGF4 Nukleinsäuren. Dies sind:
i) Die Verwendung einer für Slit1 oder MEGF4 codierenden Nukleinsäure und/oder eines Slit1 oder MEGF4 Peptids oder Proteins zur Detektion von Krebs oder zur Detektion eines Risikos der Erkrankung an Krebs, wobei eine Gewebeprobe (des betroffenen Organes, eine beliebige Gewebeprobe bei der Detektion von Metastasen aus u.g. Krebsarten) auf Transkription oder Übertranskription von Slit1 oder MEGF4 RNA oder auf Transkription oder Überexpression eines Slit1 oder MEGF4 Proteins untersucht wird, wobei vorzugsweise eine an für Slit1 oder MEGF4 codierende Nukleinsäure oder eine an Slit1 oder MEGF4 Protein oder Peptid bindende Detektorsubstanz, vorzugsweise enthaltend eine Reportergruppe, verwendet wird, wobei Bindung besagter Nukleinsäure und/oder besagten Proteins oder Peptids an die Detektorsubstanz halbquantitativ oder quantitativ detektiert wird, wobei die Krebserkrankung vorzugsweise aus der Gruppe bestehend aus "Prostatakrebs, Brustkrebs, Leberkrebs, Ovarkrebs, Colonkrebs, Pankreaskrebs und Lungenkrebs" ausgewählt ist,
ii) die Verwendung einer Slit1 oder MEGF4 RNA oder eines SLIT1 oder MEGF4 Proteins oder Peptids zum Screenen nach daran bindenden Substanzen, insbesondere prospektiven Wirkstoffen zur Inhibierung von besagter RNA oder besagtem Protein oder Peptid oder prospektiven Detektorsubstanz, wobei eine prospektive Substanz oder eine Mischung solcher prospektiver Substanzen mit besagter RNA oder besagtem Protein oder Peptid kontaktiert wird, wobei mit einem Bindungsassay Bindungsereignisse festgestellt werden, und wobei eine bindende prospektive Substanz, ggf. nach Dekonvolutierung, selektiert wird,
iii) die Verwendung einer Slit1 oder MEGF4 inhibierenden Substanz zur Herstellung einer pharmazeutischen Zusammensetzung zur Behandlung von Krebs und/oder einer Metastasenbildung aus einem Primärtumor, wobei die Krebserkrankung bzw. der Primärtumor vorzugsweise aus der Gruppe bestehend aus "Prostatakrebs, Brustkrebs, Leberkrebs, Ovarkrebs, Colonkrebs, Pankreaskrebs und Lungenkrebs" ausgewählt ist.

Eine im Rahmen der Erfindung eingesetzte Substanz kann ausgewählt sein aus der Gruppe bestehend aus:
a) Antisense-Oligonukleotide, siRNA, und Ribozyme gegen eine Nukleinsäure nach Anspruch 1,
b) an ein Peptid oder Protein nach Anspruch 2 bindendes, insbesondere nach Anspruch 5 identifiziertes, organisches Molekül mit einem Molekulargewicht unterhalb 5000, vorzugsweise unterhalb 1000, höchstvorzugsweise unterhalb 300,
c) Aptamer gegen ein Protein oder Peptid nach Anspruch 2, insbesondere identifiziert nach Anspruch 5,
d) (monoklonaler) Antikörper, insbesondere humaner oder humanisierter Antikörper gegen ein Protein oder Peptid nach Anspruch 2,
e) anti-idiotypischer nicht-humaner (monoklonale) Antikörper, generiert mittels eines Antikörpers der Unterguppe d), und
f) vorstehende Substanzen derivatisiert mit einer Reportergruppe, einem Zelltoxin, einer immunstimulierenden Komponente und/oder einem Radioisotop.

Im Falle a) kann als Ribozyme beispielsweise ein Hammerhead Ribozym eingesetzt werden. Die Ribozym-Schnittstelle wird mit der Maßgabe ausgewählt, dass durch die Aktivität des Ribozymes die Expression des Proteins entweder unterbunden wird, oder eine inaktive Form bzw. ein inaktives Fragment des Proteins exprimiert wird. Beides läßt sich beispielsweise dadurch ermitteln, dass in einem Zollsystem, in welchem ein erfindungsgemäßes Protein auf definiertem Niveau exprimiert wird, dieses Zellsystem mit einem oder mehreren für definierte Schnittstellen modelliertes Ribozym kontaktiert wird und das Expressionsniveau bestimmt bzw. die biologische Aktivität des exprimierten Proteins. Dies wird dann verglichen mit einer Negativprobe bzw. den Ergebnissen ohne Kontaktierung und Ribozyme werden selektiert, die zu niedrigerer Expression oder Aktivität führen. Entsprechend kann im Falle der siRNA oder der antisense Nukleinsäuren vorgegangen werden. Im Falle a) sind alle Sequenzen brauchbar.

Im Falle b) können chemische Stoffbibliotheken eingesetzt werden, um nach bindenden Substanzen zu screenen. Eine Validierung bindender Substanzen für therapeutische Zwecke kann durch Bestimmung der biologischen Aktivität des Proteins in einem Zellsystem mit und ohne Kontaktierung und Vergleich der erhaltenen Ergebnisse erfolgen. Für therapeutische Zwecke ausgewählt werden dann solche Stoffe, die zu einer reduzierten biologischen Aktivität führen. Es ist auch möglich, dass im Rahmen eines erfindungsgemäßen Screening Verfahren an Stelle der Bindung die biologische Aktivität bestimmt wird; dann ist eine Validierung im vorstehenden Sinne zugleich mit dem Screening erfolgt. Biologische Aktivität läßt sich beispielsweise dadurch bestimmen, dass natürliche Assoziationspartner des Proteins bestimmt und deren Vorkommen und Form (z.B. Monomer/Dimer/Heterodimer) untersucht werden. Es lassen sich auch weiter downstream in einer Stoffwechselkaskade entstehende Substanzen als Indikator verwenden; diese lassen sich beispielsweise dadurch identifizieren, dass zuvor Zellkomponenten analysiert werden für die das Protein exprimierende Zelle und ein Vergleich durchgeführt wird mit gleichen Zellen, in welchen jedoch die Expression gentechnisch deletiert ist. Es können auch können Mimikry-Verbindungen abgeleitet werden, welche in der Lage sind, spezifisch das funktionell aktive Zentrum der Enzymdomänen-enthaltenden Proteine zu binden und in ihrer biologischen Aktivität zu inhibieren. Sollte eine Mimikry-Verbindung in der Lage sein, spezifisch die Peptide zu binden, ohne es in ihrer biologischen Funktion zu inhibieren, kann es trotzdem zur Lösung des technischen und medizinischen Problems beitragen. Es kann als guter Binder mit einem Zelltoxin derivatisiert werden, um somit in einem Targeting Approach die Zielzellen abzutöten. Ein solches Zelltoxin kann ein klassisches Chemotherapeutikum aber auch ein Radioisotop sein. Ebenfalls können solche Peptid-spezifische Mimikry-Verbindungen, mit einer Reportergruppe derivatisiert, als Verbindung in der Tumordiagnostik verwendet werden.

Geeignete Aptamere (c) lassen sich unschwer beispielsweise mittels des wohlbekannten SELEX Verfahren identifizieren, wobei das erfindungsgemäße Protein als Target eingesetzt wird. Einsetzbar sind alle Sequenzen.

Antikörper (d), insbesondere monoklonale Antikörper, können in üblicher Weise durch Immunisierung eines nichtmenschlichen Säugetiers mit einem erfindungsgemäßen Protein, einer erfindungsgemäßen Nukleinsäure (z.B. cDNA), einer ein erfindungsgemäßes Protein konstitutiv exprimierenden Zelle (Krebszelle oder beispielsweise mit einer erfindungsgemäßen Nukleinsäure transfizierte Zelle, wie COS oder NIH3T3), oder mittels recombinant hergestelltem erfindungsgemäßem Protein oder Peptid, beispielsweise in E.coli oder Eukaryontenzellen (z.B. Insektenzellen) exprimiert, erhalten werden. Monoklonale Antikörper sind durch übliche Selektion und Etabilierung von Hybridomzellen erhältlich. Auch kann die Phage Display Technologie zur Generierung der Antikörper eingesetzt werden. Für den Fall (d) ist ergänzend folgendes anzumerken. Ausgehend von einer erfindungsgemäßen Sequenz kann ein Antikörper in humaner und/oder humanisierter Form abgeleitet werden, welcher in der Lage ist, spezifisch das funktionell aktive Zentrum der Proteine zu binden und in deren biologischer Aktivität zu inhibieren. Ebenfalls kann ein solcher Peptide-spezifischer Antikörper, mit einer Reportergruppe derivatisiert, als Verbindung in der Tumordiagnostik verwendet werden.

Im Falle der anti-idiotypischen Antikörper (e) sind diese dadurch erzeugbar, dass mittels eines erfindungsgemäßen Antikörpers, welcher nicht notwendigerweise die biologische Aktivität des erfindungsgemäßen Proteins beeinflussen muss, in einem nicht-menschliche Säugetier ein zweiter anti-idiotypischer (monoklonaler) Antikörper generiert wird. Dieser anti-idiotypische Antikörper täuscht dann bei Applikation in humane Zellen dem humanen Immunsystem ein Bild des Zielmoleküls vor und wird aufgrund seiner nichthumanisierten Form als körperfremdes Epitop erkannt. Der Mensch bildet folglich natürlicherweise Antikörper gegen den anti-idiotypischen Antikörper und somit auch gegen das Protein bzw. gegen das Protein exprimierende Zellen. Diese Variante der Erfindung ist ausschließlich für therapeutische Zwecke verwendbar. Ausgehend von erfindungsgemäßen Sequenzen können monoklonale Maus/Ratten Antikörper abgeleitet werden. Die monoklonalen Antikörper Ab1 müssen nicht notwendiger Weise in der Lage sein, spezifisch das funktionell aktive Zentrum der Proteine zu binden und in deren biologischer Aktivität zu inhibieren. Die monoklonalen Antikörper Ab1 werden im zweiten Schritt verwendet, um zweite anti-idiotypische maus-monoklonale Antikörper aAB1 zu generieren. Die monoklonalen aAB1 stellen die Verbindung zur Lösung des technischen und medizinischen Problems dar, in dem diese dem humanen Immunsystem ein Image des Antigens "vertäuscht" und durch seine nicht-humanisierte Form als körperfremdes Epitop erkannt wird. Der humane Körper bildet zwangsläufig eigene Antikörper gegen aAB1 und somit gegen die das Zielprotein exprimierenden Tumorzellen. Während sich die aAB1 Antikörper aussschliesslich zur Behandlung der Tumorerkrankung eignen, sind die Ab1 Antikörper ausschliesslich für die diagnostische Problemlösung zu verwenden.

Im Falle f) können ausgehend von erfindungsgemäßen Nukleinsäuresequenzen und/oder den Aminosäuresequenzen Antikörper und/oder Mimikry-Verbindungen mit hoher spezifischen Bindungsaktivität isoliert werden. Solche Substanzen werden dann in einem zweiten Schritt mit Zelltoxinen und/oder Radioisotopen derivatisiert und zum Targeting der Tumorzellen eingesetzt.

Die Erfindung betrifft weiterhin ein Verfahren zur Diagnose einer Krebserkrankung, wobei eine erfindungsgemäße Detektorsubstanz in der Ausführungsform mit einer Reportergruppe in zu untersuchendes Gewebe (welches im Organismus verbieben oder daraus entnommen sein kann) appliziert wird, wobei das zu untersuchende Gewebe dann einer Detektionsverfahrenstufe unterworfen wird, welche sensitiv für die Reportergruppe ist, und wobei im Fall der Detektion eines definierten Mindestwertes der Reportergruppe im Gewebe das Gewebe als Tumorzellen enthaltend qualifiziert wird, sowie ein Verfahren zur Behandlung einer Krebserkrankung, vorzugsweise einer Krebserkrankung ausgewählt aus der Gruppe bestehend aus "Prostatakrebs, Brustkrebs, Leberkrebs, Ovarkrebs, Colonkrebs, Pankreaskrebs und Lungenkrebs", wobei eine erfindungsgemäße pharmazeutische Zusammensetzung in einer physiologisch wirksamen Dosis und üblicher galenischer Herrichtung einem Patienten dargereicht wird.

Die Erfindung beruht auf der Erkenntnis, daß Slit1 bzw. MEGF4 differentiell in Tumorgewebe, insbesondere Prostataoder Brusttumorgewebe, exprimiert wird, i.e. in diesen Geweben ist die Expression höher, verglichen mit normalen Zellen gleichen Gewebes. Dies erlaubt es einerseits, Slit1 bzw. MEGF4 als Marker zur Identifizierung von Tumorzellen in verdächtigem Gewebe, beispielsweise in der Prostata oder der Brust, zu nutzen. Auf der anderen Seite bietet die Inhibierung von Slit1 bzw. MEGF4, insbesondere auch bei lokaler Applikation, die Möglichkeit, in die Tumorspezifischen Slit1 bzw. MEGF4 Assoziationen mit anderen Prozessen in den Tumorzellen einzugreifen und somit letztendlich den tumorzellenspezifisch veränderten Stoffwechsel zu stören und zu einem Absterben oder zumindest einer Wachstumshemmung der Tumorzellen beizutragen bzw. die Metastasierung zu inhibieren. Ensprechendes gilt im Falle der Metastasierung, da Slit1 bzw. MEGF4 die SDF-1alpha induzierte, CXCR4-mediierte Chemotaxis zu inhibieren vermag.

Im Rahmen der Erfindung kann es sich empfehlen, im Vorfeld einer Behandlung mit einer erfindungsgemäßen pharmazeutischen Zusammensetzung eine Probe aus einem Gewebe, welches als Tumorgewebe mit anderen Methoden identifiziert ist, zu entnehmen und die Gewebeprobe auf Expression bzw. Überexpression von Slit1 bzw. MEGF4 zu untersuchen. Alternativ kann mit einer erfindungsgemäßen Detektorsubstanz zur Diagnose in vivo auf Slit1 bzw. MEGF4 Abhängigkeit getestet werden. Wird eine Expression bzw. Überexpression von Slit1 bzw. MEGF4 gegenüber Normalgewebe gleichen Typs festgestellt, so ist die Anwendung der erfindungsgemäßen pharmazeutischen Zusammensetzung indiziert.

Handelt es sich bei dem Tumor um einem Typus, bei welchem Tumorzellen Slit1 bzw. MEGF4 exprimieren, Normalzellen gleichen Gewebetyps jedoch nicht oder nur schwach, so ist es besonders bevorzugt, wenn die an Slit1 bzw. MEGF4 bindende Substanz zusätzlich eine zytotoxische und/oder immunstimulierende Komponente trägt. Dies führt dann letztendlich dazu, dass praktisch ausschließlich Tumorzellen getötet, sei es durch die Zytotoxizität, sei es durch Angriff durch das stimulierte Immunsystem, werden, während Normalzellen in dem Gewebe praktisch vollständig erhalten bleiben. In dieser Ausführungsform braucht die bindende Substanz selbst nicht inhibierend auf Slitl bzw. MEGF4 zu wirken, da diese bindende Substanz dann lediglich als Marker funktionieren muß, welcher die Komponenten zu Ziel-Tumorzellen trägt. Im Falle des Einsatzes einer zytotoxischen und/oder immunstimulierenden Komponente kann es sich besonders empfehlen, wenn die pharmazeutische Zusammensetzung zur lokalen Applikation in Tumorzellen enthaltendem Gewebe hergerichtet ist, beispielsweise zur Injektion.

Mit erfindungsgemäßen Substanzen, insbesondere Peptiden, Proteinen, ist es schließlich möglich eine Immuntherapie im Wege der Vaccination mit Epitopen des Slit1 durchzuführen.

### Definitionen.

Im Rahmen dieser Beschreibung wird die Bezeichnung Slit1 bzw. MEGF4 für alle humanen Isoformen, bekannt oder neu, auf Nukleinsäuren- oder Aminosäurenbasis, verwendet. Mit diesen Begriffen mit umfaßt sind auch die im Rahmen dieser Beschreibung offenbarten kurzen Sequenzen, welche aus den Isoformen stammen, beispielsweise Immunisierungssequenzen. Weiterhin mit umfaßt sind auch Homologe, wobei die Homologie zumindest 80%, vorzugsweise mehr als 90%, höchstvorzugsweise mehr als 95%, beträgt. Im Falle der Nukleinsäuresequenzen sind auch komplementäre oder allelische Varianten mit umfaßt. Weiterhin sind Sequenzen umfaßt, welche lediglich Teilsequenzen der explizit offenbarten Sequenzen, beispielsweise ein Exon oder mehrere Exons, oder komplementärer Sequenzen hierzu darstellen, mit der Maßgabe, daß diese Teilsequenzen im Falle der Nukleinsäuren eine für eine Hybridisierung mit einer erfindungsgemäßen Nukleinsäure hinreichende Länge, zumindest 50 Basen, aufweisen und im Falle der Proteine bzw. Peptide mit zumindest gleicher Affinität an ein protein- oder peptidspezifisches Zielmolekül binden. Weiterhin sind alle mit erfindungsgemäßen Nukleinsäuren hybridisierende Nukleinsäuren umfaßt, nämlich solche, die unter stringenten Bedingungen (z.B. 5°C bis 25°C unterhalb der Aufschmelztemperatur; siehe ergänzend J.M. Sambrook et al., A laboratory manual, Cold Spring Harbor Laboratory Press (1989) und E.M. Southern, J Mol Biol, 98:503ff (1975)) hybridisieren. Es versteht sich, daß die Erfindung auch Expressionskassetten umfaßt, i.e. eine oder mehrere der erfindungsgemäßen Nukleinsäuresequenzen mit mindestens einer Kontroll- oder regulatorischen Sequenz. Eine solche Expressionskassette kann auch eine Sequenz für ein bekanntes Protein umfassen, wobei im Zuge der Translation ein Fusionsprotein aus einem bekannten Protein und einem erfindungsgemäßen Protein oder Peptid entsteht. Ebenso sind auch antisense Sequenzen zu den vorstehenden Nukleinsäuresequenzen umfaßt. Schließlich sind RNA sowie damit korrelierende DNA und umgekehrt umfaßt, ebenso wie genomische DNA als auch korrelierte cDNA und umgekehrt.

Im Zusammenhang mit erfindungsgemäßen Verwendungen umfassen die Begriffe der Slit1/MEGF4 Nukleinsäuren oder Protein bzw. Peptide neben den Volllängen der offenbarten Sequenzen (siehe auch vorstehender Absatz) auch Teilsequenzen hieraus, und zwar mit einer Mindestlänge von 12 Nukleotiden, vorzugsweise 30 bis 90 Nukleotiden, im Falle der Nukleinsäuren und einer Mindestlänge von 4 Aminosäuren, vorzugsweise 10 bis 30 Aminosäuren, im Falle der Peptide oder Proteine.

Die Begriffe der Detektion und/oder der Behandlung von Krebs, beispielsweise Prostatakrebs und/oder Brustkrebs, umfassen auch die Detektion und/oder Behandlung von Metastasen aus Primärtumoren in sonstigen Geweben. Der Begriff der Behandlung umfaßt auch die Prophylaxe.

Als Inhibitor ist eine Verbindung oder Substanz bezeichnet, welche entweder die Bildung von Slit1 bzw. MEGF4 inhibiert oder gebildetes Slit1 bzw. MEGF4 in der Aktivität reduziert, bezogen auf die Slit1 bzw. MEGF Aktivität in Abwesenheit des Inhibitors. Insofern kann ein Inhibitor einerseits eine Substanz sein, welche in der Entstehungskaskade von Slit1 bzw. MEGF4 inhibierend eingreift. Auf der anderen Seite kann ein Inhibitor eine Substanz sein, welche mit gebildetem Slit1 bzw. MEGF4 eine Bindung eingeht, und zwar dergestalt, dass weitere physiologische Wechselwirkungen mit endogenen Substanzen zumindest reduziert sind.

Mimikry-Moleküle sind Verbindungen, die den variablen Bereich, insbesondere den Bindungsbereich eines Antikörpers, nachbilden und an gleicher Stelle eines Zielmoleküls binden, wie der zu Grunde liegende Antikörper.

Der Begriff der Antikörper umfaßt polyklonale Antikörper, monoklonale Antikörper, nicht-humane, humane und humanisierte Antikörper, anti-idiotypische Antikörper, sowie Phage-Display-Antikörper, aber auch chimäre Antikörper sowie spezifische Fragmente der leichten und/oder der schweren Kette des variablen Bereiches zu Grunde liegender Antikörper vorstehender Art. Die Herstellung bzw. Gewinnung solcher Antikörper mit vorgegebenen Immunogenen ist dem Durchschnittsfachmann wohl vertraut und braucht nicht näher erläutert zu werden. Weiterhin umfaßt der Begriff der Antikörper bispezifische Antikörper. Bispezifische Antikörper kombinieren eine definierte Immunzellaktivität mit einer spezifischen Tumorzellerkennung, wodurch Tumorzellen getötet werden. Ein bispezifischer Antikörper bindet einerseits an ein Auslösemolekül der Immun-Effektorzelle (z.B. CD3, CD16, CD64) und andererseits an Antigene der Tumorzielzelle.

Die galenische Herrichtung einer erfindungsgemäßen pharmazeutischen Zusammensetzung kann in fachüblicher Weise erfolgen. Als Gegenionen für ionische Verbindungen kommen beispielsweise Na⁺, K⁺, Li⁺ oder Cyclohexylammonium infrage. Geeigente feste oder flüssige galenische Zubereitungsformen sind beispielsweise Granulate, Pulver, Dragees, Tabletten, (Mikro-) Kapseln, Suppositorien, Sirupe, Säfte, Suspensionen, Emulsionen, Tropfen oder injizierbare Lösungen (i.v., i.p., i.m.) sowie Präparate mit protrahierter Wirkstoff-Freigabe, bei deren Herstellung übliche Hilfsmittel wie Trägerstoffe, Spreng-, Binde-, Überzugs-, Quellungs-, Gleit- oder Schmiermittel, Geschmacksstoffe, Süßungsmittel und Lösungsvermittler, Verwendung finden. Als Hilfsstoffe sei Magnesiumcarbonat, Titandioxyd, Lactose, Mannit und andere Zucker, Talcum, Milcheiweiß, Gelatine, Stärke, Zellulose und ihre Derivate, tierische und pflanzliche Öle wie Lebertran, Sonnenblumen-, Erdnuss- oder Sesamöl, Polyethylenglycole und Lösungsmittel, wie etwa steriles Wasser und ein- oder mehrwertige Alkohole, beispielsweise Glycerin, genannt. Eine erfindungsgemäße pharmazeutische Zusammensetzung ist dadurch herstellbar, dass mindestens ein erfindungsgemäß verwendeter Slit1/MEGF4-Inhibitor in definierter Dosis mit einem pharmazeutisch geeigneten und physiologisch verträglichen Träger und ggf. weiteren geeigneten Wirk-, Zusatz- oder Hilfsstoffen mit definierter Inhibitordosis gemischt und zu der gewünschten Darreichungsform hergerichtet ist.

Tumorzellen exprimieren Slitl bzw. MEGF4 differenziell, wenn Normalzellen des gleichen Gewebetyps dieses nicht exprimieren. Tumorzellen überexprimieren Slit1 bzw. MEGF4 spezifisch bzw. differenziell, wenn Slit1 bzw. MEGF4 im Vergleich zu Normalzellen des gleichen Gewebes höher, beispielsweise zumindest in doppelter Menge, exprimiert wird.

Zytotoxische Komponenten bzw. Gruppen sind Verbindungen, welche direkt oder indirekt Apoptose einleiten bzw. zu Nekrose führen oder zumindest wachstumshemmend wirken. Solche Gruppen bzw. Verbindungen können neben Radioisotopen (z.B. 188Re, 213Bi, 99mTc, 90Y, 131J, 177Lu) insbesondere Zytostatika sein, welche in der Tumortherapie eingesetzt werden. Beispiele hierfür sind: Alkylantien (z.B. Mechlorethamin, Ifosfamid, Chlorambucil, Cyclophosphamid, Melphalan, Alkylsulfonate, Busulphan, Nitrosoharnstoffe, Carmustin, Lomustin, Semustin, Triazene, Dacarbazin), Antimetaboliten (z.B. Folsäure-Antagonisten, Methotrexat, Pyrimidin-Analoga, Fluoruracil, Fluordesoxyuridin, Cytarabin/ Gemcitabin, Purin-Analoga, Mercaptopurin), Mitosehemmer (z.B. Vincaalkaloide, Voncristin, Vinblastin, Paclitaxal, Docetaxel, Protaxel), Epipodophyllotoxine (z.B. Etoposid, Teniposid), Antibiotika (z.B. Dactinomycin, Daunorubicin, Idarubicin, Anthracycline, Bleomycin, L-Asparaginase), Platinkomplexverbindungen (z.B. Cisplatin), Hormone und verwandte Verbindungen (z.B. Nebennierenrindensteroide, Aminogluthetimid, Gestagene, Östrogene, Androgene, Antiöstrogene, Tamoxifen, Steriodanaloga, Flutamid). Bei Bindung einer solchen Verbindung mit einer an Slit1 bzw. MEGF bindenden Substanz erfolgt die Kopplung dergestalt, daß die Affinität zu Slitl bzw. MEGF um nicht mehr als 90%, vorzugsweise 50%, bezogen auf die Substanz ohne zytostatische Gruppe, reduziert ist und die zytostatische Wirkung der Gruppe um nicht mehr als 90%, vorzugsweise 50%, bezogen auf die Verbindung ohne Substanz, reduziert ist.

Eine immunstimulierende Komponente ist meist ein Protein oder ein wirksamer Bestandteil hiervon, welches Zellen des Immunsystems stimuliert. Beispiele hierfür sind: Zytokine, wie M-CSF, GM-CSF, G-CSF, Interferone, wie IFN-alpha, -beta, -gamma, Interleukine wie IL-1 bis -16 (außer -8), human LIF, Chemokine wie Rantes, MCAF, MIP-1-alpha, -beta, NAP-1 und IL-8.

Eine Reportergruppe ist ein Atom, Molekül oder eine Verbindung, welche in Verbindung mit einem hierauf abgestellten Assay den Nachweis der Reportergruppe und der somit mit der Reportergruppe verbundenen Verbindung oder Substanz ermöglicht. Beispiele für Reportergruppen und hiermit assoziierte Detektionsmethoden sind: 32P-Labeling und Intensitätsmessung mittels Phosphoimager. Viele weitere Beispiele, beispielsweise Fluoreszenzdetektion, sind dem Durchschnittsfachmann bekannt und bedürfen nicht der detaillierten Aufzählung.

Eine an Slit1 bzw. MEGF4 bindende Substanz kann eine Substanz sein, welche ein Slit1- bzw. MEGF4-Protein oder eine Slit1- bzw. MEGF4-RNA bindet.

Im Rahmen der vorstehenden Definition gegenüber dem engen Wortsinn erweiterte Begriffsbestimmungen umfassen auch die bestimmten Begriffe im engen Wortsinn.

### Beispiele.

Im Folgenden wird die Erfindung anhand von lediglich bevorzugte Ausführungsformen darstellenden Beispielen näher erläutert. Es zeigen:
- Fig. 1:: Chipanalyse zur differenziellen Expression von Slit1 bzw. MEGF4 in Prostatatumorgeweben aus 54 Patienten,
- Fig. 2:: Taqman®-Analyse zur differenziellen Expression von Slit1 bzw. MEGF4 in Prostatatumorgeweben aus 14 Patienten,
- Fig. 3:: Western Blots zu mittels erfindungsgemäßer Antiseren detektiertem Slit1 bzw. MEGF4,
- Fig. 4:: IHC-Nachweis von Slit1 Überexpression in Prostata Tumorzellen.
- Fig. 5:: Facs Analyse zur Lokalisierung des Slit1 bzw. MEGF4 Proteins,
- Fig. 6:: 5 verschiedene Hammerhead Ribozyme, welche Slit1 bzw. MEGF4 bzw. tSlit1 (Seq.-ID 5) mRNA schneiden,
- Fig. 7:: Erkennung von rekombinantem humanem SLIT aus stabil transfizierten HEK293-Zellen mit dem Antikörper prt87 B2,

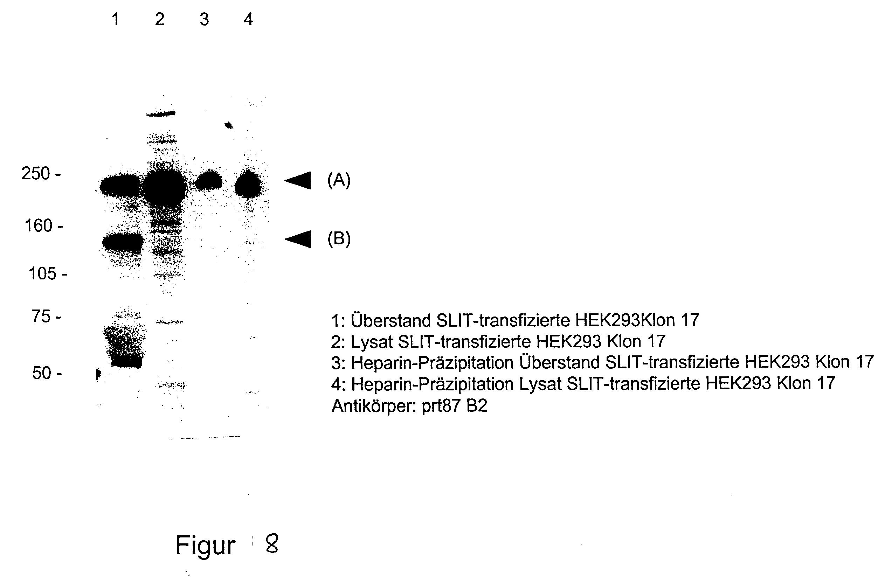

- Fig. 8:: Nachweis der Bindung von SLIT1 an Heparin-Agarose,
- Figuren 9 - 14:: Aufreinigung von SLIT1,
- Fig. 15:: graphische Darstellung von verkürzten Peptid-Sequenzen inklusive Domänen.

### Beispiel 1: Überexpression in Prostatatumor

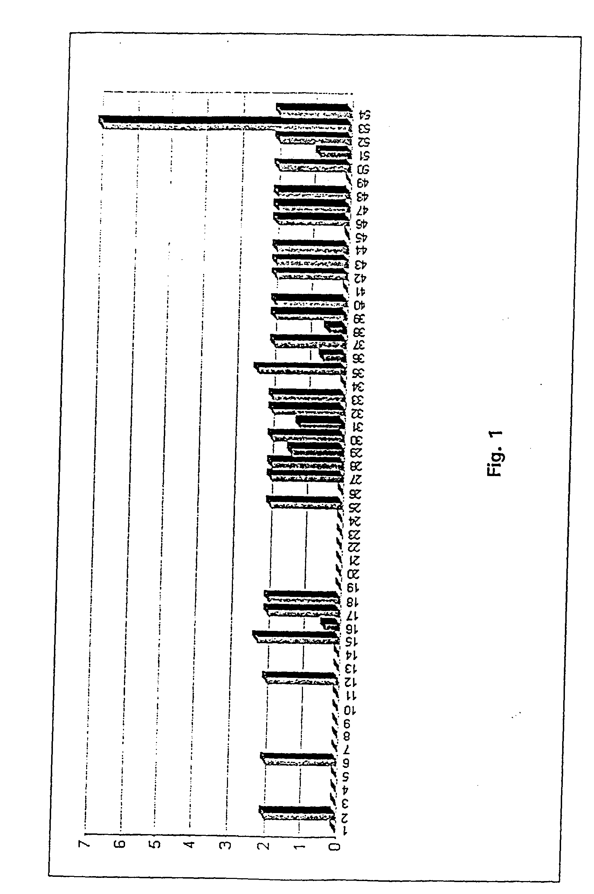

Gepaartes Tumor- und Normalgewebe wurde im Wege der Lasermicrodissektion von 54 Patienten entnommen. Die daraus präparierte RNA wurde mit Digoxygenin gelabelt und auf einen DNA Chip mit Hilfe der Affymetrix Technologie hybridisiert. Die Ergebnisse der Analyse des Chips sind in der Figur 1 dargestellt (Ordinate: Expressionsverhältnis Tumor-/Normalgewebe; Abzisse: 54 Patienten mit Prostatatumor) . Man erkennt, daß in 26 von 54 Patienten keine differenzielle Expression, in 2 von 54 eine Herunterregulierung und in 26 von 54 eine Hochregulierung von Slit1/MEGF4 festgestellt wurde (Faktor ≥ 2).

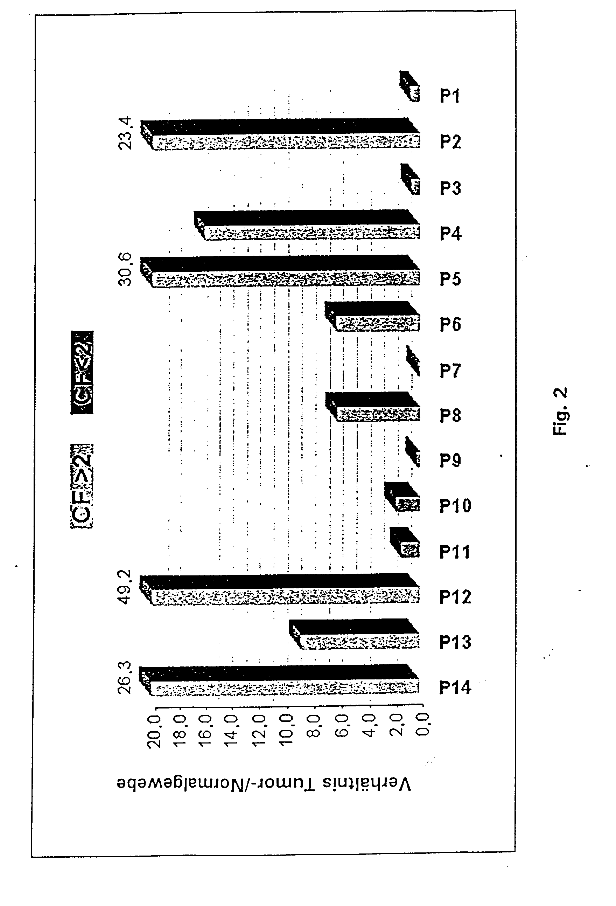

Dieser Befund wurde mit Hilfe der Taqman® Analyse überprüft. Gepaartes Tumor- und Normalgewebe von 14 Patienten wurde im Wege der Laser-Microdissektion gewonnen. Die daraus präparierte RNA wurde revers transkribiert. Die so erhaltene cDNA wurde als Templat in quantitativer PCR eingesetzt. Die Ergebnisse sind in der Figur 2 dargestellt (Ordinate wie in Fig. 1; Abzisse: 14 Patienten mit Protatatumor). Keine differenzielle Expression von Slit1/MEGF4 wurde in 6 von 14 Patienten und eine Hochregulierung der Transcription in 8 von 14 festgestellt.

Damit ist belegt, daß die Expression von Slit1/MEGF4 in mindestens 50% der Prostatatumoren hochreguliert ist, so daß sich Slit1/MEGF4 zur Detektion von Prostatatumoren eignet, und daß ein physiologischer Zusammenhang zwischen der Hochregulierung und dem Krankheitsbefund besteht und folglich eine Hemmung von Slit1/MEGF4 zur Behandlung der Tumorerkrankung geeignet ist.

### Beispiel 2: Nachweis von Slit1/MEGF4 mittels Antikörpern

Polyklonale Antikörper (AB) wurden gegen humanes Slit1 Protein (Seq.-ID 9; AB-a) und gegen humane SLIT1/MEGF4 Proteine (Seq.-ID 10; AB-b), konjugiert mit einem Trägerprotein, in Kaninchen gezogen und mit den spezifischen immobilisierten Peptiden affinitätsgereinigt.

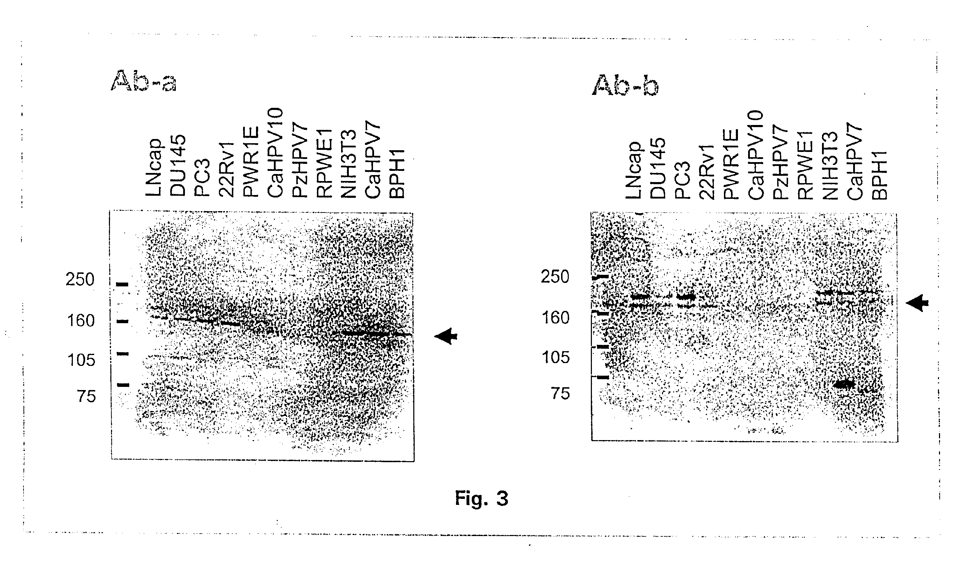

Mit den vorstehenden AB wurden Western Blots durchgeführt zum Nachweis von Slit1 und/oder MEGF4 in Prostatatumorund nicht-malignen normalen Prostatepithelzelllinien sowie in Brusttumorzelllinien. Hierzu wurden Lysate aus den ganzen Zellen (wc) oder der Membranfraktion (mem) präpariert, denaturiert und separiert mittels SDS-PAGE Gelelektrophorese. Nach dem Blotten der getrennten Proteine auf eine Membran und Blockierung unspezifischer Proteinwechselwirkungen wurde die Membran mit AB-a und/oder AB-b Antiseren über Nacht bei 4°C inkubiert. Mit einer Anti-Kaninchen-gekoppelten Meerrettichperoxidase als sekundären Antikörper wurden die spezifischen Slit1 und/oder MEGF4 Banden detektiert. Die Ergebnisse sind in der Figur 3 dargestellt. Mit AB-a erfolgte die Detektion in allen Zelllinien bei einem Molekulargewicht von ca. 190 kD. Mit AB-b wurden sowohl Slit1 (ca. 190 kD) als auch MEGF4 (ca. 180 oder 210 kD) sichtbar. Die Spezifität der detektierten Proteine wurde mit nicht dargestellten Competitionsexperimenten verifiziert unter Zugabe spezifischer und unspezifischer Peptide bei der Inkubation mit den AB.

Neben der in der Figur 3 gezeigten Expression von Slit1/MEGF4 Protein in Prostatatumorzelllinien wurde in nicht gezeigten Experimenten Expression in den Brusttumorzelllinien MaTu und MDA 231 bzw. MDA-MB-231 gefunden.

### Beispiel 3: Immunhistochemischer Nachweis von Prostatatumorzellen.

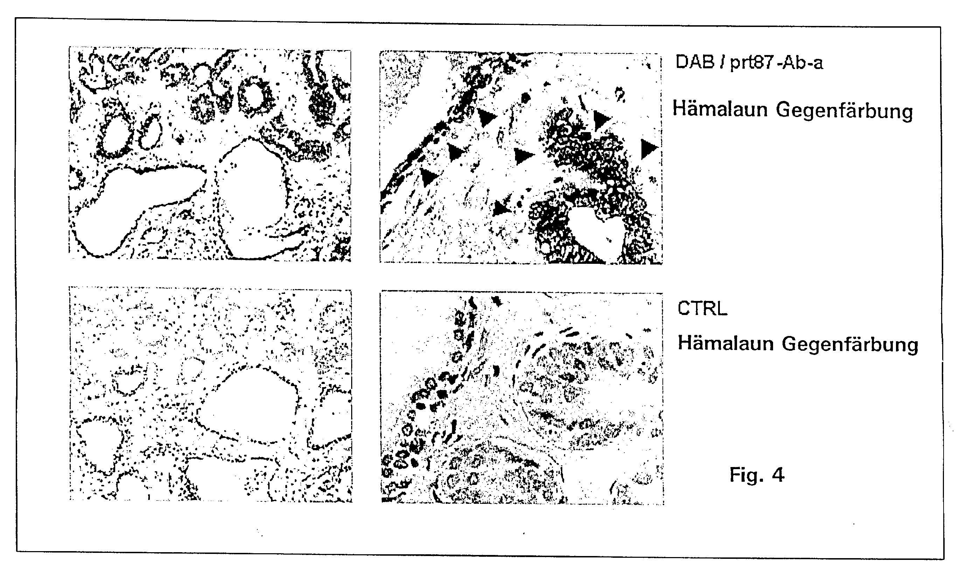

Gewebeproben aus Prostattumorgewebe wurden mit AB-a als primären Antikörper, einem biotinyliertem sekundären anti-Kaninchen Antikörper und einer Streptavidin-gekoppelten Meerrettichperoxidase inkubiert und die Färbung erfolgte mit DAB als chromogenem Substrat (braune Färbung). Die Gegenfärbung erfolgte mit Hemalaun-Lösung (blaue Färbung). Es waren maligne und normale Epithelzellen unterscheidbar, wobei die malignen Zellen eine starke Färbung, i.e. hohen Slit1 Gehalt, aufwiesen, während die normalen Epithelzellen nur moderat gefärbt waren. Die Schnitte sind in der Figur 4 gezeigt, ebenso wie die durchgeführten Negativkontrollen ohne AB-a.

### Beispiel 4

In diesem Beispiel wurde die Lokalisierung von Slit1 bzw. MEGF4 untersucht. Hierzu wurden PC3 (Prostat-Tumorzelllinie) Zellen einer Facs Analyse unterzogen unter Einsatz von AB-a und AB-b für Slit1 sowie für Slit1/MEGF4.

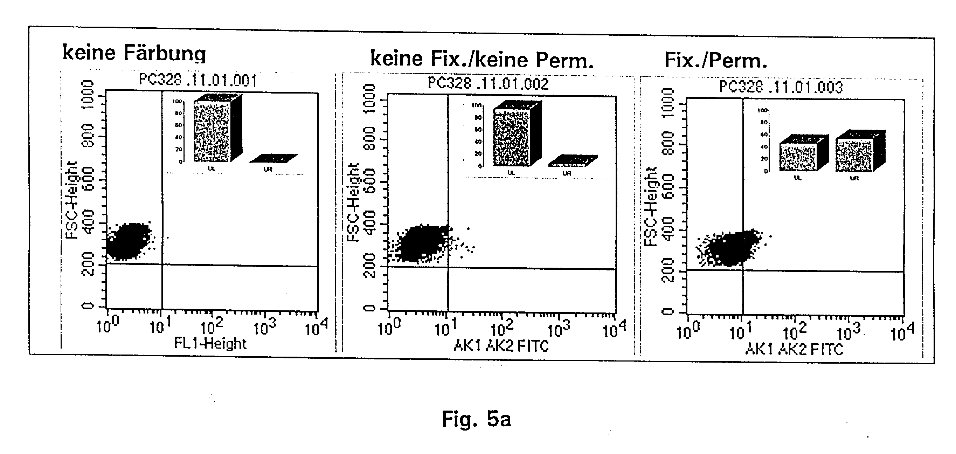

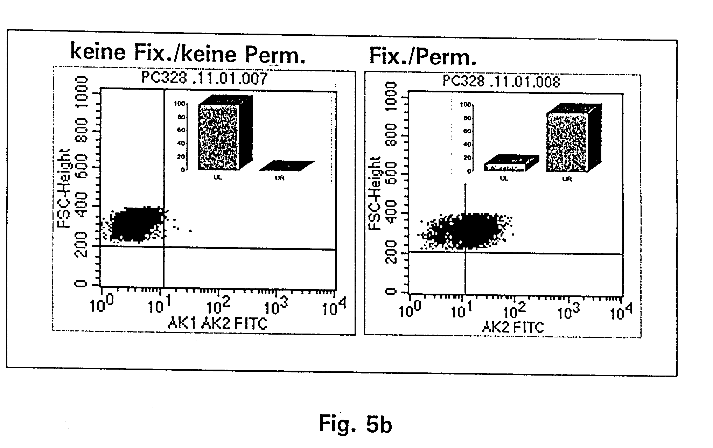

In der Figur 5a sind die Ergebnisse für AB-a und in der Fig. 5b für AB-b dargestellt. Linksseitig erkennt man, daß auf der Zellmembran von lebenden, intakten Zellen kein Slit1/MEGF4 nachweisbar ist. Nach der Permeabilisierung erfolgt dagegen eine Detektion der intracellular lokalisierten Proteine (rechts Seite).

### Beispiel 5: RNA-Inhibitoren

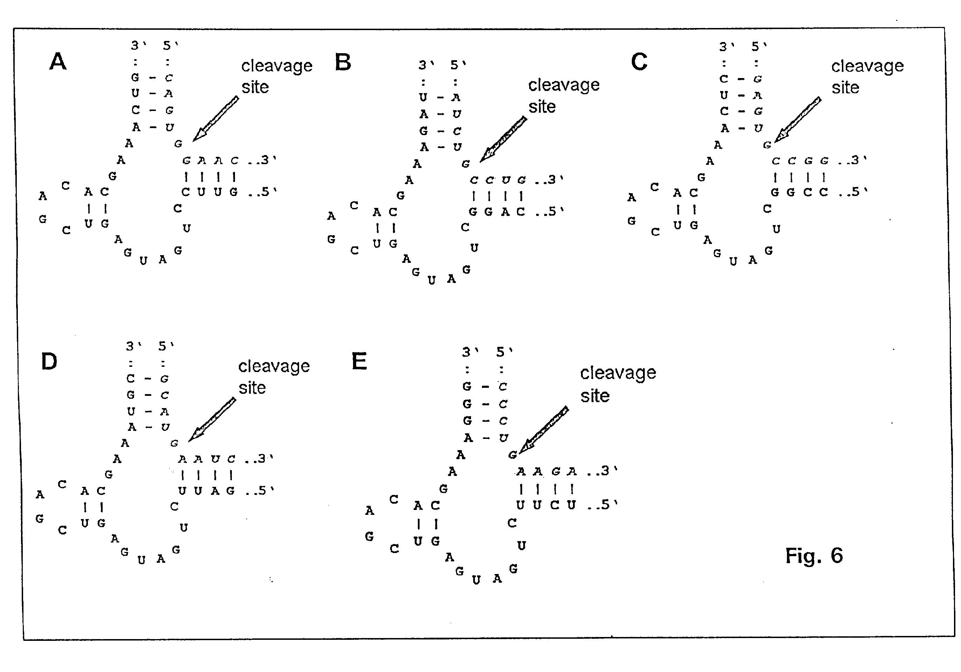

In der Figur 6 sind verschiedene Hammerhead Ribozyme dargestellt, die Slit1 bzw. MEGF4 an den dargestellten Stellen schneiden und so die Aktivität eventueller Translationsprodukte inhibieren oder zumindest deren Quantität reduzieren. Die zugehörigen Sequenzen der Figurenteile a bis e sind Seq.-ID 20 bis 24.

Die Sequenzen Seq.-ID 15 und 16 sind antisense Sequenzen für sowohl Slit1 als auch MEGF4 RNA. Die Sequenzen Seq.-ID 17, 18 und 19 sind antisense Sequenzen für Slit1, MEGF4 und tSlit1, respektive.

### Beispiel 6: Antikörpererkennung von rekombinatem Slit

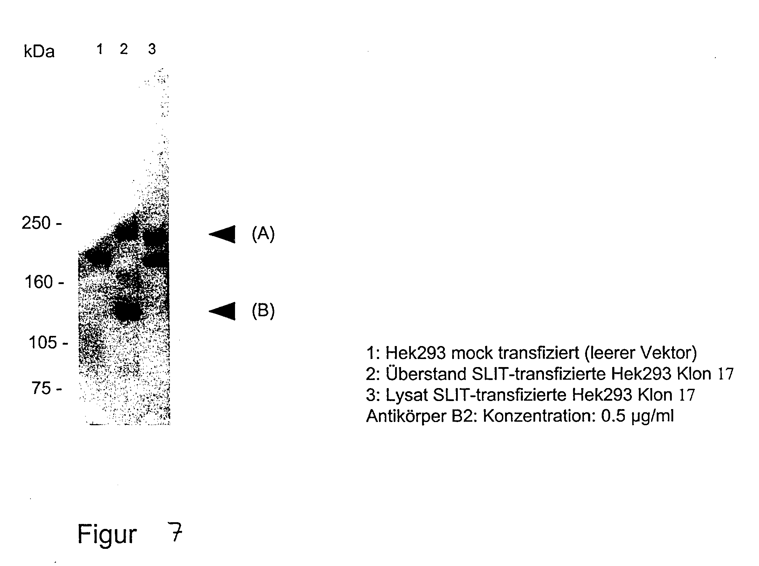

HEK293-Zellen wurden auf fachübliche Weise stabil mit einer Slit Nukleinsäure transfiziert. Gegen das entsprechende Protein wurden auf fachübliche Weise Antikörper prt87 B2 Antikörper generiert. In der Figur 7 erkennt man, dass Slit sowohl im Überstand der kultivierten HEK293-Zellen als auch im Lysat nachweisbar ist (A). Der leichte Laufhöhenunterschied in den Spuren 2 und 3 ist auf die unterschiedlichen Pufferbedingungen im Lysat bzw. Überstand bei der Elektrophorese zurückzuführen. Im Überstand ist ein kleines Protein von ca. 140 kDa detektierbar (B), welches einem hypothetischen Spaltprodukt von Slit entsprechen könnte. Die Ergebnisse zeigen, dass der Antikörper Slit spezifisch erkennt. Slit wird in den Zellkulturüberstand sektretiert und an der potentiellen Spaltstelle geschnitten.

### Beispiel 7: Bindung von Slit an Heparin Agarose.

Überstand bzw. Lysat von Zellen aus Beispiel 6 wurde mit Heparin-Agarose inkubiert. Nach dem Waschen der Beads wurde mit SDS-haltigem Puffer das gesamte an den Beads gebundene Protein eluiert und auf ein Gel aufgetragen. Slit wurde mit dem Antikörper aus Beispiel 6 nachgewiesen. Sowohl aus dem Lysat als auch aus dem Überstand läßt sich das komplette Slit1-Molekül (A) mit Heparin anreichern. Die verkürzte Form aus dem Überstand (B) bindet nicht an Heparin, was in Übereinstimmung mit der vorhergesagten Spaltstelle ist.

### Beispiel 8: Isolierung bzw. Reinigung von Slit.

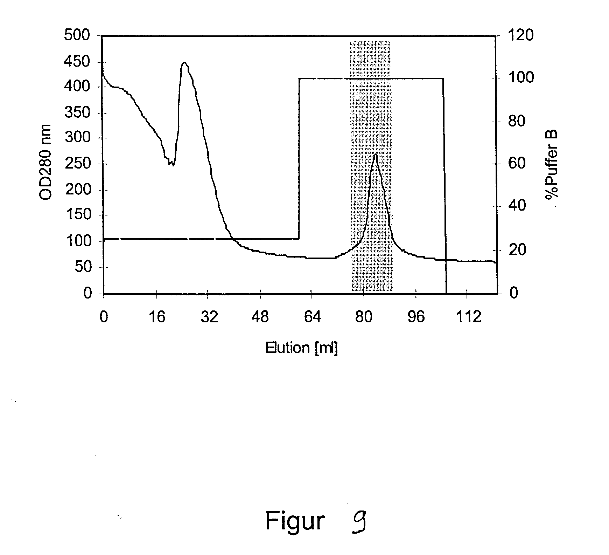

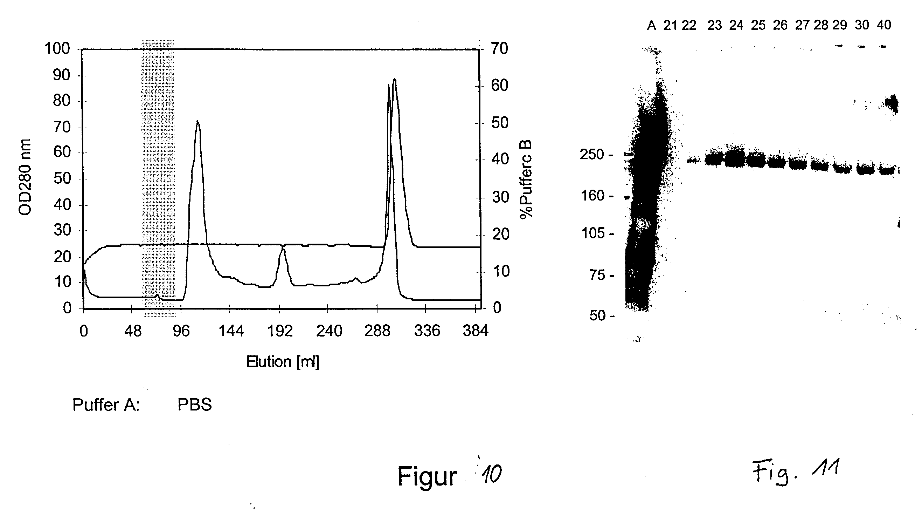

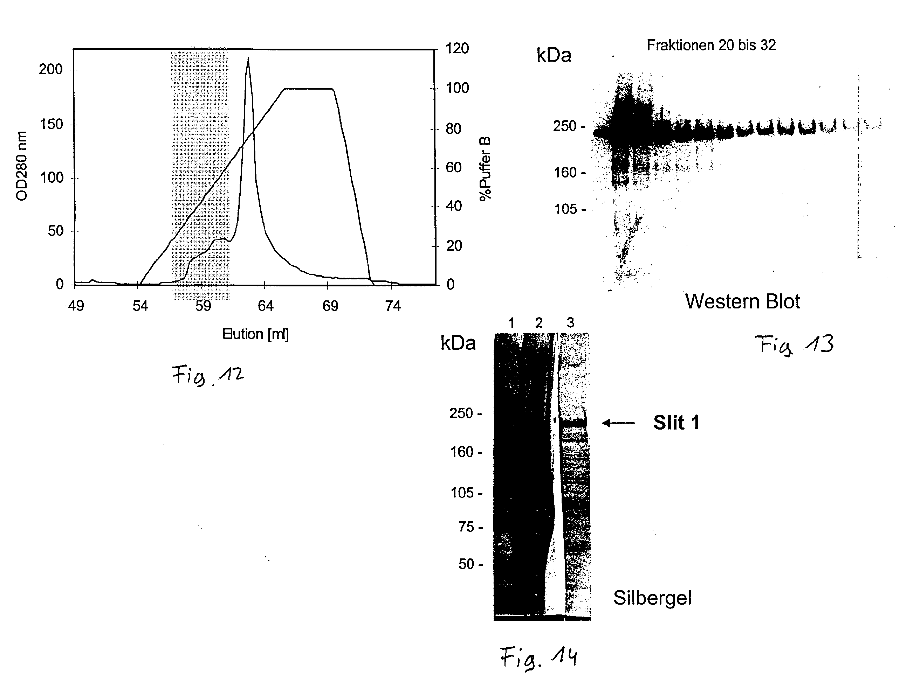

Zellen aus Beispiel 6 wurden in DMEM mit 10% Serum kultiviert und es wurden 2000 ml Kulturüberstand gewonnen. Dieser wurde über 50 kDa Cut-off Membran auf 200 ml aufkonzentriert. Hiervon wurden Proben auf eine 20 ml Heparin-Affinitätschromatographie Säule gegeben, welche mit 75% Puffer A und 25% Puffer B gefahren wurde. Slit1 wurde dann mit 100% Puffer B von der Säule eluiert. Die Fraktionen 11-14 (hellgrau, siehe Fig. 9) wurden vereinigt und auf 7 ml eingeengt. Die weitere Reinigung erfolgte über eine Gelchromatographie-Säule (Superdex200 26/60-Säule), wobei die Elution mit 1 ml/min erfolgte, wozu auf die Figur 10 verwiesen wird. Die Analyse erfolgte mit einem Westernblot mit dem Antikörper aus Beispiel 6 (Fig. 11). Die Fraktionen 21 bis 28 (siehe Fig. 10) wurden vereinigt und auf 2 M Urea eingestellt (Endvolumen 50 ml). Diese Probe wurde nach Dialyse direkt für QA-CIM-Disk (lonenaustauschchromatographie, 350 µl) verwendet, wozu auf die Fig. 12 verwiesen wird. Die Elution erfolgte mit einer ansteigenden Konzentration des Puffer B (NaCl-Gradient). Die Fraktionen 20-32 wurden in einem Western Blot (Fig. 13) aufgetrennt und Slit1 wurde mit dem Antikörper aus Beispiel 6 nachgewiesen. Daraufhin wurden die Slit1 enthaltenden Fraktionen 20-25 vereinigt und in einem Silbergel (Fig. 14) analysiert. Im Ergebnis wird Slit1 in gereinigter Form und mit dem zu erwartenden Molekulargewicht nachgewiesen.

### Beispiel 9: biologische Aktivität von Slit Fragmenten

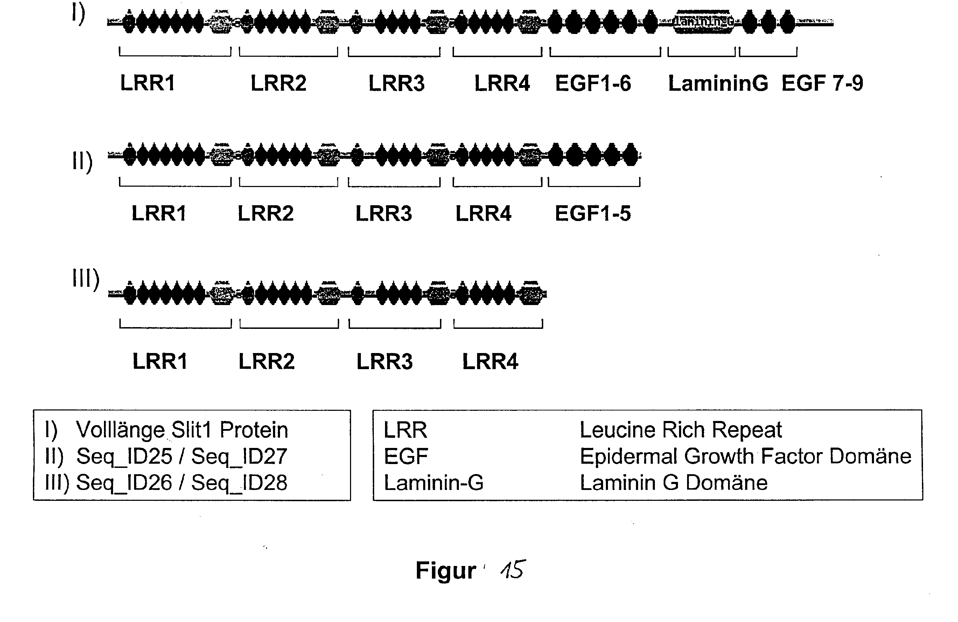

In der Fig. 15 sind Pfam Darstellungen des Slit1 Vollänge Proteins (I) sowie von 2 N-terminalen Fragnementen gemäß der Sequenzen 25 bis 28 (II und III) dargestellt. LLR steht für Leucine Rich Repeat. EGF steht für Epidermal Growth Factor Domäne. Laminin-G steht für diese Domäne. Das Fragment II ist gemäß Beispiel 6 (Bande B) nachweisbar und anscheinend das Ergebnis einer endogenen proteolytischen Spaltung. Es umfasst die LRR 1-4 und die ersten 5 EGF. Da diese Proteindomänen autonom faltend sind, wird auch das rekombinant Fragment II eine normale Faltung und vollständige biologische Aktivität aufweisen. Enstprechendes gilt für das Fragment III mit den LRR 1-4 mit seiner biologischen Aktivität als Chemorepellent.

## Patentansprüche

1. Nukleinsäure, insbesondere isolierte Nukleinsäure, codierend für eine Slit1 oder MEGF4 Isoform oder ein Fragment hiervon, enthaltend eine Nukleinsäuresequenz mit einer Sequenz gemäß Seq.-ID 2, 3, 4, 5, 25, oder 26, oder bestehend hieraus.

2. Slit1 oder MEGF4 Peptid oder Protein, insbesondere isoliert, enthaltend eine Aminosäurensequenz codiert durch eine Nukleinsäure nach Anspruch 1 oder bestehend hieraus, oder gemäß Seq.-ID 7, 8, 9, 10, 11, 12, 13, 14, 27, oder 28, oder bestehend hieraus, wobei das Protein oder Peptid vorzugsweise funktional in der Ausbildung und Funktion neuraler Gewebe von Säugetieren ist.

3. Verwendung einer für Slit1 oder MEGF4 codierenden Nukleinsäure und/oder eines Slit1 oder MEGF4 Peptids, insbesondere nach Anspruch 1 oder 2, zur Detektion von Krebs oder zur Detektion eines Risikos der Erkrankung an Krebs, wobei eine Gewebeprobe auf Transkription oder Übertranskription von Slit1 oder MEGF4 RNA oder auf Expression oder Überexpression eines Slit1 oder MEGF4 Proteins untersucht wird, wobei die Krebserkrankung vorzugsweise aus der Gruppe bestehend aus "Prostatakrebs, Brustkrebs, Leberkrebs, Ovarkrebs, Colonkrebs, Pankreaskrebs und Lungenkrebs" ausgewählt ist.

4. Verwendung nach Anspruch 3, wobei eine an für Slit1 oder MEGF4 codierende Nukleinsäure oder eine an Slit1 oder MEGF4 Protein oder Peptid bindende Detektorsubstanz, vorzugsweise enthaltend eine Reportergruppe, verwendet wird, wobei Bindung besagter Nukleinsäure und/oder besagten Proteins oder Peptids an die Detektorsubstanz halbquantitativ oder quantitativ detektiert wird.

5. Verwendung einer Slit1 oder MEGF4 DNA, RNA oder eines SLIT1 oder MEGF4 Proteins oder Peptids, insbesondere nach Anspruch 1 oder 2, und/oder einer ein solches Protein oder Peptid exprimierenden Zelle zum Screenen nach daran bindenden Substanzen, insbesondere prospektiven Wirkstoffen zur Inhibierung von besagter DNA oder RNA oder besagtem Protein oder Peptid oder nach prospektiven Detektorsubstanzen, wobei eine prospektive Substanz oder eine Mischung solcher prospektiver Substanzen mit besagter DNA, RNA oder besagtem Protein oder Peptid kontaktiert wird, wobei mit einem Bindungsassay Bindungsereignisse festgestellt werden, und wobei eine bindende prospektive Substanz, ggf. nach Dekonvolutierung, selektiert wird.

6. Verwendung einer Slit1 oder MEGF4 inhibierenden oder daran bindenden Substanz, insbesondere identifiziert nach Anpruch 5, zur Herstellung einer pharmazeutischen Zusammensetzung zur Behandlung von Krebs und/oder einer Metastasenbildung aus einem Primärtumor, wobei die Krebserkrankung bzw. der Primärtumor vorzugsweise aus der Gruppe bestehend aus "Prostatakrebs, Brustkrebs, Leberkrebs, Ovarkrebs, Colonkrebs, Pankreaskrebs und Lungenkrebs" ausgewählt ist.

7. Verwendung nach Anspruch 6, wobei die Substanz ausgewählt ist aus der Gruppe bestehend aus:
a) Antisense-Oligonukleotide, siRNA und Robozyme gegen eine Nukleinsäure nach Anspruch 1,
b) an ein Peptid oder Protein nach Anspruch 2 bindendes, insbesondere nach Anspruch 5 identifiziertes, organisches Molekül mit einem Molekulargewicht unterhalb 5000, vorzugsweise unterhalb 1000, höchstvorzugsweise unterhalb 300,
c) Aptamer gegen ein Protein oder Peptid nach Anspruch 2, insbesondere identifiziert nach Anspruch 5,
d) (monoklonaler) Antikörper, insbesondere humaner oder humanisierter Ansitkörper, gegen ein Protein oder Peptid nach Anspruch 2,
e) anti-idiotypischer nicht-humaner (monoklonaler) Antikörper, generiert mittels eines Antikörpers der Unterguppe d), und
f) vorstehende Substanzen derivatisiert mit einer Reportergruppe, einem Zelltoxin, einer immunstimulierenden Komponente und/oder einem Radioisotop.

8. Verwendung nach Anspruch 7, wobei die Substanz eine Mimikryverbindung eines Antikörpers gegen ein MEGF4 Peptid oder Protein, insbes. gemäß Anspruch 2, ist.

9. Verwendung nach Anspruch 7, wobei die Substanz ein Aptamer, eine antisense RNA gemäß einer der Nukleinsäuresequenzen Seq.-ID 15 bis 19, oder ein Ribozym gemäß einer der Nukleinsäuresequenzen Seq.-ID 20 bis 24, ist.

10. Verwendung nach einem der Ansprüche 6 bis 9, wobei die Substanz zusätzlich eine zytotoxische und/oder immunstimulierende Komponente trägt.

11. Verwendung nach einem der Ansprüche 6 bis 10, wobei die pharmazeutische Zusammensetzung zur lokalen Applikation in Tumorzellen enthaltendem Gewebe hergerichtet ist.

12. Verfahren zur Diagnose einer Krebserkrankung, wobei eine Detektorsubstanz nach Anspruch 6 bis 11 in der Ausführungsform mit einer Reportergruppe in zu untersuchendes Gewebe in vitro oder in vivo appliziert oder mit einer Blutserumprobe kontaktiert wird, wobei das zu untersuchende Gewebe bzw. die Blutserumprobe dann einer Detektionsverfahrenstufe unterworfen wird, welche sensitiv für die Reportergruppe ist, und wobei im Fall der Detektion eines definierten Mindestwertes der Reportergruppe in der Probe Gewebe eines zugeordneten Patienten als Tumorzellen enthaltend qualifiziert wird.

13. Verfahren zur Behandlung einer Krebserkrankung, vorzugsweise einer Krebserkrankung ausgewählt aus der Gruppe bestehend aus "Prostatakrebs, Brustkrebs, Leberkrebs, Ovarkrebs, Colonkrebs, Pankreaskrebs und Lungenkrebs", wobei eine pharmazeutische Zusammensetzung nach einem der Ansprüche 6 bis 11 in einer physiologisch wirksamen Dosis einem Patienten dargereicht wird.

14. Vektor enthaltend eine Nukleinsäure nach Anspruch 1.

15. Zelle, insbesondere ausgewählt aus der Gruppe bestehend aus "HEK293, Sf9 und High Five Insektenzellen", transfiziert mit einer Nukleinsäure nach Anspruch 1 bzw. einem Vektor nach Anspruch 14.


SEQUENCE LISTING

<110> metaGen Pharmaceuticals GmbH
      Mennerich, Detlev
<120> Slit-1/MEGF4 Isoformen
<130> MET/EP/0319
<150> DE 10223246.6
<151> 2002-05-21
<160> 28
<170> PatentIn version 3.1

<210> 1
<211> 5095
<212> DNA
<213> Homo sapiens
<400> 1
gcgaaacggc agaggagccg agccccctcc gcccaaggcg ccctccctcc gtccgcgcac 60

aggcgccgtc gcttggagga ccaaggtgcc tcccagcccg caggggcgcc gcgcgcaagc 120

ccgcgggctc ttcggtggct ctgccccggg actgcacctg gaggcggccc cggacgggga 180

tggtcagcgg ctgctgccgt ctggctcgcg agcgggacgc tgtgagggca ccatggcgct 240

gactcccggg tgggggtcct cggcggggcc ggtccggccg gagctctggc tgctgctgtg 300

ggcagccgcg tggcgcctgg gtgcctcggc gtgccccgcc ctctgcacct gcaccggaac 360

cacggtggac tgccacggca cggggctgca ggccattccc aagaatatac ctcggaacac 420

cgagcgcctg gaactcaatg gcaacaacat cactcggatc cataagaatg actttgcggg 480

gctcaagcag ctgcgggtgc tgcagctgat ggagaaccag attggagcag tggaacgtgg 540

tgcttttgat gacatgaagg agctggagcg gctgcgactg aaccgaaacc agctgcacat 600

gttaccggaa ctgctgttcc agaacaacca ggctttgtca agactggact tgagtgagaa 660

cgccatccag gccatcccca ggaaagcttt tcggggagct acggacctta aaaatttaca 720

gctggacaag aaccagatca gctgcattga ggaaggggcc ttccgtgctc tgcgggggct 780

ggaggtgctg accctgaaca acaacaatat caccaccatc cccgtgtcca gcttcaacca 840

tatgcccaag ctacggacct tccgcctgca ctccaaccac ctgttttgcg actgccacct 900

ggcctggctc tcgcagtggc tgaggcagcg gccaaccatc gggctcttca cccagtgctc 960

gggcccagcc agcctgcgtg gcctcaatgt ggcagaggtc cagaagagtg agttcagctg 1020

ctcaggccag ggagaagcgg ggcgcgtgcc cacctgcacc ctgtcctccg gctcctgccc 1080

ggccatgtgc acctgcagca atggcatcgt ggactgtcgt ggaaaaggcc tcactgccat 1140

cccggccaac ctgcccgaga ccatgacgga gatacgcctg gagctgaacg gcatcaagtc 1200

catccctcct ggagccttct caccctacag aaagctacgg aggatagacc tgagcaacaa 1260

tcagatcgct gagattgcac ccgacgcctt ccagggcctc cgctccctga actcgctggt 1320

cctctatgga aacaagatca cagacctccc ccgtggtgtg tttggaggcc tatacaccct 1380

acagctcctg ctcctgaatg ccaacaagat caactgcatc cggcccgatg ccttccagga 1440

cctgcagaac ctctcactgc tctccctgta tgacaacaag atccagagcc tcgccaaggg 1500
cactttcacc tccctgcggg ccatccagac tctgcacctg gcgcagaacc ctttcatttg 1560
cgactgtaac ctcaagtggc tggcagactt cctgcgcacc aatcccatcg agacgagtgg 1620
tgcccgctgt gccagtcccc ggcgcctcgc caacaagcgc atcgggcaga tcaagagcaa 1680
gaagttccgg tgctcagcca aagagcagta cttcattcca ggcacggagg attaccagct 1740
gaacagcgag tgcaacagcg acgtggtctg tccccacaag tgccgctgtg aggccaacgt 1800
ggtggagtgc tccagcctga agctcaccaa gatccctgag cgcatccccc agtccacggc 1860
agaactgcga ttgaataaca atgagatttc catcctggag gccactggga tgtttaaaaa 1920
acttacacat ctgaagaaaa tcaatctgag caacaacaag gtgtcagaaa ttgaagatgg 1980
ggccttcgag ggcgcagcct ctgtgagcga gctgcaccta actgccaacc agctggagtc 2040
catccggagc ggcatgttcc ggggtctgga tggcttgagg accctaatgc tgcggaacaa 2100
ccgcatcagc tgcatccaca acgacagctt cacgggcctg cgcaacgtcc ggctcctctc 2160
gctctacgac aaccagatca ccaccgtatc cccaggagcc ttcgacaccc tccagtccct 2220
ctccacactg aatctcctgg ccaacccttt caactgcaac tgccagctgg cctggctagg 2280
aggctggcta cggaagcgca agatcgtgac ggggaacccg cgatgccaga accctgactt 2340
tttgcggcag attcccctgc aggacgtggc cttccctgac ttcaggtgtg aggaaggcca 2400
ggaggagggg ggctgcctgc cccgcccaca gtgcccacag gagtgcgcct gcctggacac 2460
cgtggtccga tgcagcaaca agcacctgcg ggccctgccc aagggcattc ccaagaatgt 2520
cacagaactc tatttggacg ggaaccagtt cacgctggtt ccgggacagc tgtctacctt 2580
caagtacctg cagctcgtgg acctgagcaa caacaagatc agttccttaa gcaattcctc 2640
cttcaccaac atgagccagc tgaccactct gatcctcagc tacaatgccc tgcagtgcat 2700
cctgcctttg gccttccagg gactccgctc cctgcgcctg ctgtctctcc acggcaatga 2760
catctccacc ctccaagagg gcatctttgc agacgtgacc tccctgtctc acctggccat 2820
tggtgccaac cccctatact gtgactgcca cctccgctgg ctgtccagct gggtgaagac 2880
tggctacaag gaaccgggca ttgctcgttg tgctgggccc caggacatgg agggcaagct 2940
gctcctcacc acgcctgcca agaagtttga atgccaaggt cctccaacgc tggctgtcca 3000
ggccaagtgt gatctctgct tgtccagtcc gtgccagaac cagggcacct gccacaacga 3060
cccccttgag gtgtacaggt gcgcctgccc cagcggctat aagggtcgag actgtgaggt 3120
gtccctggac agctgttcca gtggcccctg tgaaaatggg ggcacctgcc atgcacagga 3180
gggcgaggat gccccgttca cgtgctcctg tcccaccggc tttgaaggac caacctgtgg 3240
ggtgaacaca gatgactgtg tggatcatgc ctgtgccaat gggggcgtct gtgtggatgg 3300

tgtgggcaac tacacctgcc agtgccccct gcagtatgag ggaaaggcct gtgagcagct 3360
ggtggacttg tgctctccgg atctgaaccc atgtcaacac gaggcccagt gtgtgggcac 3420
cccggatggg cccaggtgtg agtgcatgcc aggttatgca ggtgacaact gcagtgagaa 3480
ccaggatgac tgcagggacc accgctgcca gaatggggcc cagtgtatgg atgaagtcaa 3540
cagctactcc tgcctctgtg ctgagggcta cagtggacag ctctgtgaga tccctcccca 3600
tctgcctgcc cccaagagcc cctgtgaggg gactgagtgc cagaatgggg ccaactgtgt 3660
ggaccagggc aacaggcctg tgtgccagtg cctcccaggc ttcggtggcc ctgagtgtga 3720
gaagttgctc agtgtcaact ttgtggatcg ggacacttac ctgcagttca ctgacctgca 3780
aaactggcca cgggccaaca tcacgttgca ggtctccacg gcagaggaca atgggatcct 3840
tctgtacaac ggggacaacg accacattgc agttgagctg taccagggcc atgtgcgtgt 3900
cagctacgac ccaggcagct accccagctc tgccatctac agtgctgaga cgatcaacga 3960
tgggcaattc cacaccgttg agctggttgc ctttgaccag atggtgaatc tctccattga 4020
tggcgggagc cccatgacca tggacaactt tggcaaacat tacacgctca acagcgaggc 4080
gccactctat gtgggaggga tgcccgtgga tgtcaactca gctgccttcc gcctgtggca 4140
gatcctcaac ggcaccggct tccacggttg catccgaaac ctgtacatca acaacgagct 4200
gcaggacttc accaagacgc agatgaagcc aggcgtggtg ccaggctgcg aaccctgccg 4260
caagctctac tgcctgcatg gcatctgcca gcccaatgcc accccagggc ccatgtgcca 4320
ctgcgaggct ggctgggtgg gcctgcactg tgaccagccc gctgacggcc cctgccatgg 4380
ccacaagtgt gtccatgggc aatgcgtgcc cctcgacgct ctttcctaca gctgccagtg 4440
ccaggatggg tactcggggg cactgtgcaa ccaggccggg gccctggcag agccctgcag 4500
aggcctgcag tgcctgcatg gccactgcca ggcctcaggc accaaggggg cacactgtgt 4560
gtgtgacccc ggcttttcgg gcgagctgtg tgagcaagag tccgagtgcc ggggggaccc 4620
tgtccgggac tttcaccagg tccagagggg ctatgccatc tgccagacca cgcgccccct 4680
gtcatgggtg gagtgccggg gctcgtgccc aggccagggc tgctgccagg gccttcggct 4740
gaagcggagg aagttcacct ttgagtgcag cgatggaccc tcttttgccg aggaggtgga 4800
aaagcccacc aagtgtggct gtgccctctg cgcatagcgc tgggcgtgga caggccggtg 4860
agggcgggca aggggcccca gccgctgcag cagcggagac agtcgccagc agctgggctg 4920
gggtgcaggt catcacagga cggctcctgg gcagctgggc cctcctgggt ggggtggtgc 4980
cagagcagcc ttttaaaagc aaattgcgcc atagctgggg gcagcggggg tgggcgaggc 5040
ctgagctgcg ggctgccctc tccggaagtg ccttgcacaa ataggcgctt aataa 5095

<210> 2
<211> 7910

<212>  DNA
<213>  Homo sapiens
<400>  2
gcgaaacggc agaggagccg agccccctcc gcccaaggcg ccctccctcc gtccgcgcac      60
aggcgccgtc gcttggagga ccaaggtgcc tcccagcccg caggggcgcc gcgcgcaagc     120
ccgcgggctc ttcggtggct ctgccccggg actgcacctg gaggcggccc cggacgggga     180
tggtcagcgg ctgctgccgt ctggctcgcg agcgggacgc tgtgagggca ccatggcgct     240
gactcccggg tgggggtcct cggcggggcc ggtccggccg gagctctggc tgctgctgtg     300
ggcagccgcg tggcgcctgg gtgcctcggc gtgccccgcc ctctgcacct gcaccggaac     360
cacggtggac tgccacggca cggggctgca ggccattccc aagaatatac ctcggaacac     420
cgagcgcctg gaactcaatg gcaacaacat cactcggatc cataagaatg actttgcggg     480
gctcaagcag ctgcgggtgc tgcagctgat ggagaaccag attggagcag tggaacgtgg     540
tgcttttgat gacatgaagg agctggagcg gctgcgactg aaccgaaacc agctgcacat     600
gttaccggaa ctgctgttcc agaacaacca ggctttgtca agactggact tgagtgagaa     660
cgccatccag gccatcccca ggaaagcttt tcggggagct acggacctta aaaatttaca     720
gctggacaag aaccagatca gctgcattga ggaaggggcc ttccgtgctc tgcgggggct     780
ggaggtgctg accctgaaca acaacaatat caccaccatc cccgtgtcca gcttcaacca     840
tatgcccaag ctacggacct tccgcctgca ctccaaccac ctgttttgcg actgccacct     900
ggcctggctc tcgcagtggc tgaggcagcg gccaaccatc gggctcttca cccagtgctc     960
gggcccagcc agcctgcgtg gcctcaatgt ggcagaggtc cagaagagtg agttcagctg    1020
ctcaggccag ggagaagcgg ggcgcgtgcc cacctgcacc ctgtcctccg gctcctgccc    1080
ggccatgtgc acctgcagca atggcatcgt ggactgtcgt ggaaaaggcc tcactgccat    1140
cccggccaac ctgcccgaga ccatgacgga gatacgcctg gagctgaacg gcatcaagtc    1200
catccctcct ggagccttct caccctacag aaagctacgg aggatagacc tgagcaacaa    1260
tcagatcgct gagattgcac ccgacgcctt ccagggcctc cgctccctga actcgctggt    1320
cctctatgga aacaagatca cagacctccc ccgtggtgtg tttggaggcc tatacaccct    1380
acagctcctg ctcctgaatg ccaacaagat caactgcatc cggcccgatg ccttccagga    1440
cctgcagaac ctctcactgc tctccctgta tgacaacaag atccagagcc tcgccaaggg    1500
cactttcacc tccctgcggg ccatccagac tctgcacctg gcgcagaacc ctttcatttg    1560
cgactgtaac ctcaagtggc tggcagactt cctgcgcacc aatcccatcg agacgagtgg    1620
tgcccgctgt gccagtcccc ggcgcctcgc caacaagcgc atcgggcaga tcaagagcaa    1680
gaagttccgg tgctcagcca aagagcagta cttcattcca ggcacggagg attaccagct    1740
gaacagcgag tgcaacagcg acgtggtctg tccccacaag tgccgctgtg aggccaacgt    1800

ggtggagtgc tccagcctga agctcaccaa gatccctgag cgcatccccc agtccacggc 1860
agaactgcga ttgaataaca atgagatttc catcctggag gccactggga tgtttaaaaa 1920
acttacacat ctgaagaaaa tcaatctgag caacaacaag gtgtcagaaa ttgaagatgg 1980
ggccttcgag ggcgcagcct ctgtgagcga gctgcaccta actgccaacc agctggagtc 2040
catccggagc ggcatgttcc ggggtctgga tggcttgagg accctaatgc tgcggaacaa 2100
ccgcatcagc tgcatccaca acgacagctt cacgggcctg cgcaacgtcc ggctcctctc 2160
gctctacgac aaccagatca ccaccgtatc cccaggagcc ttcgacaccc tccagtccct 2220
ctccacactg aatctcctgg ccaacccttt caactgcaac tgccagctgg cctggctagg 2280
aggctggcta cggaagcgca agatcgtgac ggggaacccg cgatgccaga accctgactt 2340
tttgcggcag attcccctgc aggacgtggc cttccctgac ttcaggtgtg aggaaggcca 2400
ggaggagggg ggctgcctgc cccgcccaca gtgcccacag gagtgcgcct gcctggacac 2460
cgtggtccga tgcagcaaca agcacctgcg ggccctgccc aagggcattc ccaagaatgt 2520
cacagaactc tatttggacg ggaaccagtt cacgctggtt ccgggacagc tgtctacctt 2580
caagtacctg cagctcgtgg acctgagcaa caacaagatc agttccttaa gcaattcctc 2640
cttcaccaac atgagccagc tgaccactct gatcctcagc tacaatgccc tgcagtgcat 2700
cccgcctttg gccttccagg gactccgctc cctgcgcctg ctgtctctcc acggcaatga 2760
catctccacc ctccaagagg gcatctttgc agacgtgacc tccctgtctc acctggccat 2820
tggtgccaac cccctatact gtgactgcca cctccgctgg ctgtccagct gggtgaagac 2880
tggctacaag gaaccgggca ttgctcgttg tgctgggccc caggacatgg agggcaagct 2940
gctcctcacc acgcctgcca agaagtttga atgccaaggt cctccaacgc tggctgtcca 3000
ggccaagtgt gatctctgct tgtccagtcc gtgccagaac cagggcacct gccacaacga 3060
cccccttgag gtgtacaggt gcgcctgccc cagcggctat aagggtcgag actgtgaggt 3120
gtccctggac agctgttcca gtggcccctg tgaaaatggg ggcacctgcc atgcacagga 3180
gggcgaggat gccccgttca cgtgctcctg tcccaccggc tttgaaggac caacctgtgg 3240
ggtgaacaca gatgactgtg tggatcatgc ctgtgccaat gggggcgtct gtgtggatgg 3300
tgtgggcaac tacacctgcc agtgccccct gcagtatgag ggaaaggcct gtgagcagct 3360
ggtggacttg tgctctccgg atctgaaccc atgtcaacac gaggcccagt gtgtgggcac 3420
cccggatggg cccaggtgtg agtgcatgcc aggttatgca ggtgacaact gcagtgagaa 3480
ccaggatgac tgcagggacc accgctgcca gaatggggcc cagtgtatgg atgaagtcaa 3540
cagctactcc tgcctctgtg ctgagggcta cagtggacag ctctgtgaga tccctcccca 3600
tctgcctgcc cccaagagcc cctgtgaggg gactgagtgc cagaatgggg ccaactgtgt 3660
ggaccagggc aacaggcctg tgtgccagtg cctcccaggc ttcggtggcc ctgagtgtga 3720

gaagttgctc agtgtcaact ttgtggatcg ggacacttac ctgcagttca ctgacctgca 3780
aaactggcca cgggccaaca tcacgttgca ggtctccacg gcagaggaca atgggatcct 3840
tctgtacaac ggggacaacg accacattgc agttgagctg taccagggcc atgtgcgtgt 3900
cagctacgac ccaggcagct accccagctc tgccatctac agtgctgaga cgatcaacga 3960
tgggcaattc cacaccgttg agctggttgc ctttgaccag atggtgaatc tctccattga 4020
tggcgggagc cccatgacca tggacaactt tggcaaacat tacacgctca acagcgaggc 4080
gccactctat gtgggaggga tgcccgtgga tgtcaactca gctgccttcc gcctgtggca 4140
gatcctcaac ggcaccggct tccacggttg catccgaaac ctgtacatca acaacgagct 4200
gcaggacttc accaagacgc agatgaagcc aggcgtggtg ccaggctgcg aaccctgccg 4260
caagctctac tgcctgcatg gcatctgcca gcccaatgcc accccagggc ccatgtgcca 4320
ctgcgaggct ggctgggtgg gcctgcactg tgaccagccc gctgacggcc cctgccatgg 4380
ccacaagtgt gtccatgggc aatgcgtgcc cctcgacgct ctttcctaca gctgccagtg 4440
ccaggatggg tactcggggg cactgtgcaa ccaggccggg gccctggcag agccctgcag 4500
aggcctgcag tgcctgcatg gccactgcca ggcctcaggc accaaggggg cacactgtgt 4560
gtgtgacccc ggcttttcgg gcgagctgtg tgagcaagag tccgagtgcc ggggggaccc 4620
tgtccgggac tttcaccagg tccagagggg ctatgccatc tgccagacca cgcgccccct 4680
gtcatgggtg gagtgccggg gctcgtgccc aggccagggc tgctgccagg gccttcggct 4740
gaagcggagg aagttcacct ttgagtgcag cgatgggacc tcttttgccg aggaggtgga 4800
aaagcccacc aagtgtggct gtgccctctg cgcatagcgc tgggcgtgga caggccggtg 4860
agggcgggca aggggcccca gccgctgcag cagcggagac agtcgccagc agctgggctg 4920
gggtgcaggt catcacagga cggctcctgg gcagctgggc cctcctgggt ggggtggtgc 4980
cagagcagcc ttttaaaagc aaattgcgcc atagctgggg gcagcggggg tgggcgaggc 5040
ctgagctgcg ggctgccctc tccggaagtg ccttgcacaa ataggcgctt aataaatatt 5100
tgttgagtga atgtgtgcgt gaggtcaggc caagaagtgc agaacgatga cacccctcct 5160
tacctgctat ctgaatctgg agaagaaaaa tgacagcctt ccaaaccaac ccttcccttt 5220
ggcctgtggc ccaggctggc ttggaactgg gtctgtggcc ccagaagcct cttacccctc 5280
tgcgggcaac catgaagtac tgtcagcctc cccgggaagc cagcctggtt cattctgctg 5340
ctacagaatc tgctggtggt aggccaggct ctggagcggg ggtgccgcct cctgctggcc 5400
agggagggtc ggacccttgc cccctgggct gactggcagc tctgcagcca cggcttggga 5460
acgaggctgt gggtggaggt ggttcttagg accaggcctc tgaatcctaa agttctagca 5520
tgactactgt agctgcgagg gcttatgtgg aggaaacagt cacaggggct gctcagggtg 5580

gcagacccca ctaaagaggg cagagggttc tttgctctag ataaacaaac atcatctgcc 5640
tccagacact ggccacagta ggagtattgg tcctgggctt ccccagccac cagtcagcca 5700
caagctgtcg gtgacctatt ggtagaggga ctgggtgtga gggtctgggc cagggtgctt 5760
gacctgggag cagctggttc agagtccttc acaccgcagg ccagtaggga gcagtggaag 5820
ggacagtgct ccaggcattg ggaagtccct gctggctcta tcactcgggg caaacttctc 5880
cccacctggg ccttgggttc ttcagctata aaatggccag aggtgggggg cgggatgact 5940
aaaggaacag tgcagactcc cccactgtgg tcttgggagg ccagaggagt tagaagacct 6000
atctatctat ctatctatct acattgatca catcaaaagt atttatgtgc ctaacccggg 6060
gctggggatt gtggacgttc tggcctaatg gacagatgtg aactcatccc agagcatcgc 6120
aggaatgacc aggatgcccg ggaagagttg agctgagtgg gggctccagc cacagacagc 6180
ggcccaggcc agggagttgc tggcaacgaa ggagccagtg gtggaagaag aagaggccct 6240
gaatatacga ttgcctgccc acgttgtctt ctcttccata cacagtgaaa atgtagaaag 6300
atggtttgtg aggccaaact gtgaatgggc taaagggagg caaagttgca ctctccttcc 6360
ccagagggct caccaagagg gcacaccccc gggggttctg gtgggcaacg ggggtgagca 6420
tgtccctgcc ctggctccct ccatctgtga ccaggaggca tggctgggtg tatgttcagg 6480
tgaggctcag agtggcattg tgtccctgtc ccctgcccag ggcagtgagg ggagcccttg 6540
atgctgatta gaaggctaga actggggtag aggtgcctgg catgtctcat gccatgggga 6600
ctcaatctag caactgtgag tcctggggtc cctgtgatgg gaagagggca gtgccctgcc 6660
caatgtggca ggtgtcctca tggcaggatc tgcccctcac cagggggctg ggatctactt 6720
gcttggagct ctgagcaagg ccacaatgcc cgcccccacc cccaagtaga ctgcagcctg 6780
ggcctcatgg ggcttctccc aggcccacat ggcatccctc tctgagtttc caggccaccg 6840
tgggaccctg cagagcatct gcaccgggct ggatagggca gaaaagctca agggcagcta 6900
gcttgcctct tccctggaag aaaggtgctc tgggactcac caaccctgag aaagatagct 6960
ttcctggcca ccaccattcc ccaccaccct ggagaagcca attcccaggc ttgaagggca 7020
ctggctggca ggaggcctct tcattctgca ggaggtggaa aggacacctg tagacaggtg 7080
atgctcaccc ctcacctggc gccatggggc tgggaggtga gcggctggca tgtttgttcc 7140
tagggagcac catgtgagct taaggctccc ctgaccggcc ccaccacatg gcccagcctc 7200
ctagcacagc agcgctgacc tcagtgcagt ctgaggattg gaatccacca tgagatgatg 7260
tgagagctgt gtgccccagg atcaactttt tctccaactt ggccatcagc cagcgagttg 7320
ctaaggacct gagtcagcac tcacgttgcc tattcacact ccgcttgaaa gtccggaagg 7380
tggctactgc aaaatcaccc ctctgagaag tcctctctcc acatcttgtc cccctttgtg 7440
aagaccccta gttcgctctg cattttaggc atgaagagat acagcagggt gcgtccggag 7500

ggagctgtgg ccttgcaaca ccactggcaa cagggccggg gctcccggtg aaggtgtcag 7560

gaagtggaaa aggctggact ttgtctcctc tttgcctgct ggtagcctaa ccgcaaaagt 7620

atctctttat acagaatact tacagattct aatatatatt tgtatttcat tttgttacag 7680

tatttttata tgttaaagtc aacatccagc gtcttgtttt gcctttcaga tgctatgtgg 7740

tcgtggcacg ttttgttggg ggtttctgta gtcgtcttgt ttggatcaac tcctagaggc 7800

tggtttagaa caggcccatg agggagctgc acctgccctg gaagtattgt tttagactat 7860

gtcgatattg tctgttgtct tccatgtgaa catgacattg agtcactctg 7910

<210> 3
<211> 5902
<212> DNA
<213> Homo sapiens
<400> 3
gcgaaacggc agaggagccg agccccctcc gcccaaggcg ccctccctcc gtccgcgcac 60

aggcgccgtc gcttggagga ccaaggtgcc tcccagcccg caggggcgcc gcgcgcaagc 120

ccgcgggctc ttcggtggct ctgccccggg actgcacctg gaggcggccc cggacgggga 180

tggtcagcgg ctgctgccgt ctggctcgcg agcgggacgc tgtgagggca ccatggcgct 240

gactcccggg tgggggtcct cggcggggcc ggtccggccg gagctctggc tgctgctgtg 300

ggcagccgcg tggcgcctgg gtgcctcggc gtgccccgcc ctctgcacct gcaccggaac 360

cacggtggac tgccacggca cggggctgca ggccattccc aagaatatac ctcggaacac 420

cgagcgcctg gaactcaatg gcaacaacat cactcggatc cataagaatg actttgcggg 480

gctcaagcag ctgcgggtgc tgcagctgat ggagaaccag attggagcag tggaacgtgg 540

tgcttttgat gacatgaagg agctggagcg gctgcgactg aaccgaaacc agctgcacat 600

gttaccggaa ctgctgttcc agaacaacca ggctttgtca agactggact tgagtgagaa 660

cgccatccag gccatcccca ggaaagcttt tcggggagct acggacctta aaaatttaca 720

gctggacaag aaccagatca gctgcattga ggaaggggcc ttccgtgctc tgcgggggct 780

ggaggtgctg accctgaaca acaacaatat caccaccatc cccgtgtcca gcttcaacca 840

tatgcccaag ctacggacct tccgcctgca ctccaaccac ctgttttgcg actgccacct 900

ggcctggctc tcgcagtggc tgaggcagcg gccaaccatc gggctcttca cccagtgctc 960

gggcccagcc agcctgcgtg gcctcaatgt ggcagaggtc cagaagagtg agttcagctg 1020

ctcaggccag ggagaagcgg ggcgcgtgcc cacctgcacc ctgtcctccg gctcctgccc 1080

ggccatgtgc acctgcagca atggcatcgt ggactgtcgt ggaaaaggcc tcactgccat 1140

cccggccaac ctgcccgaga ccatgacgga gatacgcctg gagctgaacg gcatcaagtc 1200

catccctcct ggagccttct caccctacag aaagctacgg aggatagacc tgagcaacaa 1260

tcagatcgct gagattgcac ccgacgcctt ccagggcctc cgctccctga actcgctggt 1320
cctctatgga aacaagatca cagacctccc ccgtggtgtg tttggaggcc tatacaccct 1380
acagctcctg ctcctgaatg ccaacaagat caactgcatc cggcccgatg ccttccagga 1440
cctgcagaac ctctcactgc tctccctgta tgacaacaag atccagagcc tcgccaaggg 1500
cactttcacc tccctgcggg ccatccagac tctgcacctg gcgcagaacc ctttcatttg 1560
cgactgtaac ctcaagtggc tggcagactt cctgcgcacc aatcccatcg agacgagtgg 1620
tgcccgctgt gccagtcccc ggcgcctcgc caacaagcgc atcgggcaga tcaagagcaa 1680
gaagttccgg tgctcagcca aagagcagta cttcattcca ggcacggagg attaccagct 1740
gaacagcgag tgcaacagcg acgtggtctg tccccacaag tgccgctgtg aggccaacgt 1800
ggtggagtgc tccagcctga agctcaccaa gatccctgag cgcatccccc agtccacggc 1860
agaactgcga ttgaataaca atgagatttc catcctggag gccactggga tgtttaaaaa 1920
acttacacat ctgaagaaaa tcaatctgag caacaacaag gtgtcagaaa ttgaagatgg 1980
ggccttcgag ggcgcagcct ctgtgagcga gctgcaccta actgccaacc agctggagtc 2040
catccggagc ggcatgttcc ggggtctgga tggcttgagg accctaatgc tgcggaacaa 2100
ccgcatcagc tgcatccaca acgacagctt cacgggcctg cgcaacgtcc ggctcctctc 2160
gctctacgac aaccagatca ccaccgtatc cccaggagcc ttcgacaccc tccagtccct 2220
ctccacactg aatctcctgg ccaacccttt caactgcaac tgccagctgg cctggctagg 2280
aggctggcta cggaagcgca agatcgtgac ggggaacccg cgatgccaga accctgactt 2340
tttgcggcag attcccctgc aggacgtggc cttccctgac ttcaggtgtg aggaaggcca 2400
ggaggagggg ggctgcctgc cccgcccaca gtgcccacag gagtgcgcct gcctggacac 2460
cgtggtccga tgcagcaaca agcacctgcg ggccctgccc aagggcattc ccaagaatgt 2520
cacagaactc tatttggacg ggaaccagtt cacgctggtt ccgggacagc tgtctacctt 2580
caagtacctg cagctcgtgg acctgagcaa caacaagatc agttccttaa gcaattcctc 2640
cttcaccaac atgagccagc tgaccactct gatcctcagc tacaatgccc tgcagtgcat 2700
cccgcctttg gccttccagg gactccgctc cctgcgcctg ctgtctctcc acggcaatga 2760
catctccacc ctccaagagg gcatctttgc agacgtgacc tccctgtctc acctggccat 2820
tggtgccaac cccctatact gtgactgcca cctccgctgg ctgtccagct gggtgaagac 2880
tggctacaag gaaccgggca ttgctcgttg tgctgggccc caggacatgg agggcaagct 2940
gctcctcacc acgcctgcca agaagtttga atgccaaggt cctccaacgc tggctgtcca 3000
ggccaagtgt gatctctgct tgtccagtcc gtgccagaac cagggcacct gccacaacga 3060
cccccttgag gtgtacaggt gcgcctgccc cagcggctat aagggtcgag actgtgaggt 3120
gtccctggac agctgttcca gtggcccctg tgaaaatggg ggcacctgcc atgcacagga 3180

gggcgaggat gccccgttca cgtgctcctg tcccaccggc tttgaaggac caacctgtgg 3240
ggtgaacaca gatgactgtg tggatcatgc ctgtgccaat gggggcgtct gtgtggatgg 3300
tgtgggcaac tacacctgcc agtgccccct gcagtatgag ggaaaggcct gtgagcagct 3360
ggtggacttg tgctctccgg atctgaaccc atgtcaacac gaggcccagt gtgtgggcac 3420
cccggatggg cccaggtgtg agtgcatgcc aggttatgca ggtgacaact gcagtgagaa 3480
ccaggatgac tgcagggacc accgctgcca gaatggggcc cagtgtatgg atgaagtcaa 3540
cagctactcc tgcctctgtg ctgagggcta cagtggacag ctctgtgaga tccctcccca 3600
tctgcctgcc cccaagagcc cctgtgaggg gactgagtgc cagaatgggg ccaactgtgt 3660
ggaccagggc aacaggcctg tgtgccagtg cctcccaggc ttcggtggcc ctgagtgtga 3720
gaagttgctc agtgtcaact ttgtggatcg ggacacttac ctgcagttca ctgacctgca 3780
aaactggcca cgggccaaca tcacgttgca ggtctccacg gcagaggaca atgggatcct 3840
tctgtacaac ggggacaacg accacattgc agttgagctg taccagggcc atgtgcgtgt 3900
cagctacgac ccaggcagct accccagctc tgccatctac agtgctgaga cgatcaacga 3960
tgggcaattc cacaccgttg agctggttgc ctttgaccag atggtgaatc tctccattga 4020
tggcgggagc cccatgacca tggacaactt tggcaaacat tacacgctca acagcgaggc 4080
gccactctat gtgggaggga tgcccgtgga tgtcaactca gctgccttcc gcctgtggca 4140
gatcctcaac ggcaccggct tccacggttg catccgaaac ctgtacatca acaacgagct 4200
gcaggacttc accaagacgc agatgaagcc aggcgtggtg ccaggctgcg aaccctgccg 4260
caagctctac tgcctgcatg gcatctgcca gcccaatgcc accccagggc ccatgtgcca 4320
ctgcgaggct ggctgggtgg gcctgcactg tgaccagccc gctgacggcc cctgccatgg 4380
ccacaagtgt gtccatgggc aatgcgtgcc cctcgacgct ctttcctaca gctgccagtg 4440
ccaggatggg tactcggggg cactgtgcaa ccaggccggg gccctggcag agccctgcag 4500
aggcctgcag tgcctgcatg gccactgcca ggcctcaggc accaaggggg cacactgtgt 4560
gtgtgacccc ggcttttcgg gcgagctgtg tgagcaaggt caggggcccc cctcctgacg 4620
tgccctcccc agggtccccc acaaattgct ttagcaattt gactcttcct cccagtctcg 4680
gcagcccttc tgtcccttcc cagccctgta ccatgggcta ctatggggct ttcagagtcc 4740
ctccaccctc tgggcactgc tcccaatctc tcctggccca gtttgccctg cagctccctt 4800
attcagggta tgcgcctctt ctggctgggg tctctccttg cagaagagac cacccagggt 4860
aacagctcct ggcccaacct ctgcctcttg gccccatcac ctcatgacct gggagcggca 4920
gcaggaagtc cgagggttgg ggactccttc ccaggccctc cctggcctgc ttgaccaaga 4980
tggcttctgc tggaatccgg actgctaagg ctgggcaggg agaagctgga gaaacccaga 5040

actcaggcac cttaagggtc ccctaggcta tcccctcccc agcatgaatc cttttcataa 5100

tgttattgcc cccgggccct ccagcctctc ttgcacgcct ctagcaatgg ggaccccccc 5160

tccctccagc agttcagtct gtcctggagg gccctgacgc taggaccaga ctgtaggccc 5220

tgggaggaag cttgtgcatg tcagtgggag gcggaagtat gctggggaca gaggtggggt 5280

ctgagagcca aaggatccac cccacagatg ctgccctcct cctcctcttc ctcctgcctc 5340

cctggaggca gcagctcacc catgggtgtg ccctgaggct ttctcttggt gtctgtccca 5400

tccagagtcc gagtgccggg gggaccctgt ccgggacttt caccaggtcc agaggggcta 5460

tgccatctgc cagaccacgc gccccctgtc atgggtggag tgccggggct cgtgcccagg 5520

ccagggctgc tgccagggcc ttcggctgaa gcggaggaag ttcacctttg agtgcagcga 5580

tgggacctct tttgccgagg aggtggaaaa gcccaccaag tgtggctgtg ccctctgcgc 5640

atagcgctgg gcgtggacag gccggtgagg gcgggcaagg ggccccagcc gctgcagcag 5700

cggagacagt cgccagcagc tgggctgggg tgcaggtcat cacaggacgg ctcctgggca 5760

gctgggccct cctgggtggg gtggtgccag agcagccttt taaaagcaaa ttgcgccata 5820

gctgggggca gcgggggtgg gcgaggcctg agctgcgggc tgccctctcc ggaagtgcct 5880

tgcacaaata ggcgcttaat aa 5902

<210> 4
<211> 8717
<212> DNA
<213> Homo sapiens
<400> 4
gcgaaacggc agaggagccg agccccctcc gcccaaggcg ccctccctcc gtccgcgcac 60

aggcgccgtc gcttggagga ccaaggtgcc tcccagcccg caggggcgcc gcgcgcaagc 120

ccgcgggctc ttcggtggct ctgccccggg actgcacctg gaggcggccc cggacgggga 180

tggtcagcgg ctgctgccgt ctggctcgcg agcgggacgc tgtgagggca ccatggcgct 240

gactcccggg tgggggtcct cggcggggcc ggtccggccg gagctctggc tgctgctgtg 300

ggcagccgcg tggcgcctgg gtgcctcggc gtgccccgcc ctctgcacct gcaccggaac 360

cacggtggac tgccacggca cggggctgca ggccattccc aagaatatac ctcggaacac 420

cgagcgcctg gaactcaatg gcaacaacat cactcggatc cataagaatg actttgcggg 480

gctcaagcag ctgcgggtgc tgcagctgat ggagaaccag attggagcag tggaacgtgg 540

tgcttttgat gacatgaagg agctggagcg gctgcgactg aaccgaaacc agctgcacat 600

gttaccggaa ctgctgttcc agaacaacca ggctttgtca agactggact tgagtgagaa 660

cgccatccag gccatcccca ggaaagcttt tcggggagct acggacctta aaaatttaca 720

gctggacaag aaccagatca gctgcattga ggaaggggcc ttccgtgctc tgcgggggct 780

ggaggtgctg accctgaaca acaacaatat caccaccatc cccgtgtcca gcttcaacca 840

tatgcccaag ctacggacct tccgcctgca ctccaaccac ctgttttgcg actgccacct 900
ggcctggctc tcgcagtggc tgaggcagcg gccaaccatc gggctcttca cccagtgctc 960
gggcccagcc agcctgcgtg gcctcaatgt ggcagaggtc cagaagagtg agttcagctg 1020
ctcaggccag ggagaagcgg ggcgcgtgcc cacctgcacc ctgtcctccg gctcctgccc 1080
ggccatgtgc acctgcagca atggcatcgt ggactgtcgt ggaaaaggcc tcactgccat 1140
cccggccaac ctgcccgaga ccatgacgga gatacgcctg gagctgaacg gcatcaagtc 1200
catccctcct ggagccttct caccctacag aaagctacgg aggatagacc tgagcaacaa 1260
tcagatcgct gagattgcac ccgacgcctt ccagggcctc cgctccctga actcgctggt 1320
cctctatgga aacaagatca cagacctccc ccgtggtgtg tttggaggcc tatacaccct 1380
acagctcctg ctcctgaatg ccaacaagat caactgcatc cggcccgatg ccttccagga 1440
cctgcagaac ctctcactgc tctccctgta tgacaacaag atccagagcc tcgccaaggg 1500
cactttcacc tccctgcggg ccatccagac tctgcacctg gcgcagaacc ctttcatttg 1560
cgactgtaac ctcaagtggc tggcagactt cctgcgcacc aatcccatcg agacgagtgg 1620
tgcccgctgt gccagtcccc ggcgcctcgc caacaagcgc atcgggcaga tcaagagcaa 1680
gaagttccgg tgctcagcca aagagcagta cttcattcca ggcacggagg attaccagct 1740
gaacagcgag tgcaacagcg acgtggtctg tccccacaag tgccgctgtg aggccaacgt 1800
ggtggagtgc tccagcctga agctcaccaa gatccctgag cgcatccccc agtccacggc 1860
agaactgcga ttgaataaca atgagatttc catcctggag gccactggga tgtttaaaaa 1920
acttacacat ctgaagaaaa tcaatctgag caacaacaag gtgtcagaaa ttgaagatgg 1980
ggccttcgag ggcgcagcct ctgtgagcga gctgcaccta actgccaacc agctggagtc 2040
catccggagc ggcatgttcc ggggtctgga tggcttgagg accctaatgc tgcggaacaa 2100
ccgcatcagc tgcatccaca acgacagctt cacgggcctg cgcaacgtcc ggctcctctc 2160
gctctacgac aaccagatca ccaccgtatc cccaggagcc ttcgacaccc tccagtccct 2220
ctccacactg aatctcctgg ccaacccttt caactgcaac tgccagctgg cctggctagg 2280
aggctggcta cggaagcgca agatcgtgac ggggaacccg cgatgccaga accctgactt 2340
tttgcggcag attcccctgc aggacgtggc cttccctgac ttcaggtgtg aggaaggcca 2400
ggaggagggg ggctgcctgc cccgcccaca gtgcccacag gagtgcgcct gcctggacac 2460
cgtggtccga tgcagcaaca agcacctgcg ggccctgccc aagggcattc ccaagaatgt 2520
cacagaactc tatttggacg ggaaccagtt cacgctggtt ccgggacagc tgtctacctt 2580
caagtacctg cagctcgtgg acctgagcaa caacaagatc agttccttaa gcaattcctc 2640
cttcaccaac atgagccagc tgaccactct gatcctcagc tacaatgccc tgcagtgcat 2700

cccgcctttg gccttccagg gactccgctc cctgcgcctg ctgtctctcc acggcaatga 2760
catctccacc ctccaagagg gcatctttgc agacgtgacc tccctgtctc acctggccat 2820
tggtgccaac cccctatact gtgactgcca cctccgctgg ctgtccagct gggtgaagac 2880
tggctacaag gaaccgggca ttgctcgttg tgctgggccc caggacatgg agggcaagct 2940
gctcctcacc acgcctgcca agaagtttga atgccaaggt cctccaacgc tggctgtcca 3000
ggccaagtgt gatctctgct tgtccagtcc gtgccagaac cagggcacct gccacaacga 3060
cccccttgag gtgtacaggt gcgcctgccc cagcggctat aagggtcgag actgtgaggt 3120
gtccctggac agctgttcca gtggcccctg tgaaaatggg ggcacctgcc atgcacagga 3180
gggcgaggat gccccgttca cgtgctcctg tcccaccggc tttgaaggac caacctgtgg 3240
ggtgaacaca gatgactgtg tggatcatgc ctgtgccaat gggggcgtct gtgtggatgg 3300
tgtgggcaac tacacctgcc agtgccccct gcagtatgag ggaaaggcct gtgagcagct 3360
ggtggacttg tgctctccgg atctgaaccc atgtcaacac gaggcccagt gtgtgggcac 3420
cccggatggg cccaggtgtg agtgcatgcc aggttatgca ggtgacaact gcagtgagaa 3480
ccaggatgac tgcagggacc accgctgcca gaatggggcc cagtgtatgg atgaagtcaa 3540
cagctactcc tgcctctgtg ctgagggcta cagtggacag ctctgtgaga tccctcccca 3600
tctgcctgcc cccaagagcc cctgtgaggg gactgagtgc cagaatgggg ccaactgtgt 3660
ggaccagggc aacaggcctg tgtgccagtg cctcccaggc ttcggtggcc ctgagtgtga 3720
gaagttgctc agtgtcaact ttgtggatcg ggacacttac ctgcagttca ctgacctgca 3780
aaactggcca cgggccaaca tcacgttgca ggtctccacg gcagaggaca atgggatcct 3840
tctgtacaac ggggacaacg accacattgc agttgagctg taccagggcc atgtgcgtgt 3900
cagctacgac ccaggcagct accccagctc tgccatctac agtgctgaga cgatcaacga 3960
tgggcaattc cacaccgttg agctggttgc ctttgaccag atggtgaatc tctccattga 4020
tggcgggagc cccatgacca tggacaactt tggcaaacat tacacgctca acagcgaggc 4080
gccactctat gtgggaggga tgcccgtgga tgtcaactca gctgccttcc gcctgtggca 4140
gatcctcaac ggcaccggct tccacggttg catccgaaac ctgtacatca acaacgagct 4200
gcaggacttc accaagacgc agatgaagcc aggcgtggtg ccaggctgcg aaccctgccg 4260
caagctctac tgcctgcatg gcatctgcca gcccaatgcc accccagggc ccatgtgcca 4320
ctgcgaggct ggctgggtgg gcctgcactg tgaccagccc gctgacggcc cctgccatgg 4380
ccacaagtgt gtccatgggc aatgcgtgcc cctcgacgct ctttcctaca gctgccagtg 4440
ccaggatggg tactcggggg cactgtgcaa ccaggccggg gccctggcag agccctgcag 4500
aggcctgcag tgcctgcatg gccactgcca ggcctcaggc accaaggggg cacactgtgt 4560
gtgtgacccc ggcttttcgg gcgagctgtg tgagcaaggt caggggcccc cctcctgacg 4620

tgccctcccc agggtccccc acaaattgct ttagcaattt gactcttcct cccagtctcg 4680
gcagcccttc tgtcccttcc cagccctgta ccatgggcta ctatggggct ttcagagtcc 4740
ctccaccctc tgggcactgc tcccaatctc tcctggccca gtttgccctg cagctccctt 4800
attcagggta tgcgcctctt ctggctgggg tctctccttg cagaagagac cacccagggt 4860
aacagctcct ggcccaacct ctgcctcttg gccccatcac ctcatgacct gggagcggca 4920
gcaggaagtc cgagggttgg ggactccttc ccaggccctc cctggcctgc ttgaccaaga 4980
tggcttctgc tggaatccgg actgctaagg ctgggcaggg agaagctgga gaaacccaga 5040
actcaggcac cttaagggtc ccctaggcta tcccctcccc agcatgaatc cttttcataa 5100
tgttattgcc cccggggcct ccagcctctc ttgcacgcct ctagcaatgg ggaccccccc 5160
tccctccagc agttcagtct gtcctggagg gccctgacgc taggaccaga ctgtaggccc 5220
tgggaggaag cttgtgcatg tcagtgggag gcggaagtat gctggggaca gaggtggggt 5280
ctgagagcca aaggatccac cccacagatg ctgccctcct cctcctcttc ctcctgcctc 5340
cctggaggca gcagctcacc catgggtgtg ccctgaggct ttctcttggt gtctgtccca 5400
tccagagtcc gagtgccggg gggaccctgt ccgggacttt caccaggtcc agaggggcta 5460
tgccatctgc cagaccacgc gccccctgtc atgggtggag tgccggggct cgtgcccagg 5520
ccagggctgc tgccagggcc ttcggctgaa gcggaggaag ttcacctttg agtgcagcga 5580
tgggacctct tttgccgagg aggtggaaaa gcccaccaag tgtggctgtg ccctctgcgc 5640
atagcgctgg gcgtggacag gccggtgagg gcgggcaagg ggccccagcc gctgcagcag 5700
cggagacagt cgccagcagc tgggctgggg tgcaggtcat cacaggacgg ctcctgggca 5760
gctgggccct cctgggtggg gtggtgccag agcagccttt taaaagcaaa ttgcgccata 5820
gctgggggca gcgggggtgg gcgaggcctg agctgcgggc tgccctctcc ggaagtgcct 5880
tgcacaaata ggcgcttaat aaatatttgt tgagtgaatg tgtgcgtgag gtcaggccaa 5940
gaagtgcaga acgatgacac ccctccttac ctgctatctg aatctggaga agaaaaatga 6000
cagccttcca aaccaaccct tccctttggc ctgtggccca ggctggcttg gaactgggtc 6060
tgtggcccca gaagcctctt acccctctgc gggcaaccat gaagtactgt cagcctcccc 6120
gggaagccag cctggttcat tctgctgcta cagaatctgc tggtggtagg ccaggctctg 6180
gagcgggggt gccgcctcct gctggccagg gagggtcgga cccttgcccc ctgggctgac 6240
tggcagctct gcagccacgg cttgggaacg aggctgtggg tggaggtggt tcttaggacc 6300
aggcctctga atcctaaagt tctagcatga ctactgtagc tgcgagggct tatgtggagg 6360
aaacagtcac aggggctgct cagggtggca gaccccacta aagagggcag agggttcttt 6420
gctctagata aacaaacatc atctgcctcc agacactggc cacagtagga gtattggtcc 6480

tgggcttccc cagccaccag tcagccacaa gctgtcggtg acctattggt agagggactg 6540
ggtgtgaggg tctgggccag ggtgcttgac ctgggagcag ctggttcaga gtccttcaca 6600
ccgcaggcca gtagggagca gtggaaggga cagtgctcca ggcattggga agtccctgct 6660
ggctctatca ctcggggcaa acttctcccc acctgggcct tgggttcttc agctataaaa 6720
tggccagagg tggggggcgg gatgactaaa ggaacagtgc agactccccc actgtggtct 6780
tgggaggcca gaggagttag aagacctatc tatctatcta tctatctaca ttgatcacat 6840
caaaagtatt tatgtgccta acccggggct ggggattgtg gacgttctgg cctaatggac 6900
agatgtgaac tcatcccaga gcatcgcagg aatgaccagg atgcccggga agagttgagc 6960
tgagtggggg ctccagccac agacagcggc ccaggccagg gagttgctgg caacgaagga 7020
gccagtggtg gaagaagaag aggccctgaa tatacgattg cctgcccacg ttgtcttctc 7080
ttccatacac agtgaaaatg tagaaagatg gtttgtgagg ccaaactgtg aatgggctaa 7140
agggaggcaa agttgcactc tccttcccca gagggctcac caagagggca cacccccggg 7200
ggttctggtg ggcaacgggg gtgagcatgt ccctgccctg gctccctcca tctgtgacca 7260
ggaggcatgg ctgggtgtat gttcaggtga ggctcagagt ggcattgtgt ccctgtcccc 7320
tgcccagggc agtgagggga gcccttgatg ctgattagaa ggctagaact ggggtagagg 7380
tgcctggcat gtctcatgcc atggggactc aatctagcaa ctgtgagtcc tggggtccct 7440
gtgatgggaa gagggcagtg ccctgcccaa tgtggcaggt gtcctcatgg caggatctgc 7500
ccctcaccag ggggctggga tctacttgct tggagctctg agcaaggcca caatgcccgc 7560
ccccaccccc aagtagactg cagcctgggc ctcatggggc ttctcccagg cccacatggc 7620
atccctctct gagtttccag gccaccgtgg gaccctgcag agcatctgca ccgggctgga 7680
tagggcagaa aagctcaagg gcagctagct tgcctcttcc ctggaagaaa ggtgctctgg 7740
gactcaccaa ccctgagaaa gatagctttc ctggccacca ccattcccca ccaccctgga 7800
gaagccaatt cccaggcttg aagggcactg gctggcagga ggcctcttca ttctgcagga 7860
ggtggaaagg acacctgtag acaggtgatg ctcacccctc acctggcgcc atggggctgg 7920
gaggtgagcg gctggcatgt ttgttcctag ggagcaccat gtgagcttaa ggctcccctg 7980
accggcccca ccacatggcc cagcctccta gcacagcagc gctgacctca gtgcagtctg 8040
aggattggaa tccaccatga gatgatgtga gagctgtgtg ccccaggatc aactttttct 8100
ccaacttggc catcagccag cgagttgcta aggacctgag tcagcactca cgttgcctat 8160
tcacactccg cttgaaagtc cggaaggtgg ctactgcaaa atcacccctc tgagaagtcc 8220
tctctccaca tcttgtcccc ctttgtgaag acccctagtt cgctctgcat tttaggcatg 8280
aagagataca gcagggtgcg tccggaggga gctgtggcct tgcaacacca ctggcaacag 8340
ggccggggct cccggtgaag gtgtcaggaa gtggaaaagg ctggactttg tctcctcttt 8400

gcctgctggt agcctaaccg caaaagtatc tctttataca gaatacttac agattctaat 8460

atatatttgt atttcatttt gttacagtat ttttatatgt taaagtcaac atccagcgtc 8520

ttgttttgcc tttcagatgc tatgtggtcg tggcacgttt tgttgggggt ttctgtagtc 8580

gtcttgtttg gatcaactcc tagaggctgg tttagaacag gcccatgagg gagctgcacc 8640

tgccctggaa gtattgtttt agactatgtc gatattgtct gttgtcttcc atgtgaacat 8700

gacattgagt cactctg 8717

<210> 5
<211> 767
<212> DNA
<213> Homo sapiens
<220>
<221> misc_feature
<222> (762)..(762)
<223> beliebiges Nukleotid

<400> 5
gcgaaacggc agaggagccg agccccctcc gcccaaggcg ccctccctcc gtccgcgcac 60

aggcgccgtc gcttggagga ccaaggtgcc tcccagcccg caggggcgcc gcgcgcaagc 120

ccgcgggctc ttcggtggct ctgccccggg actgcacctg gaggcggccc cggacgggga 180

tggtcagcgg ctgctgccgt ctggctcgcg agcgggacgc tgtgagggca ccatggcgct 240

gactcccggg tgggggtcct cggcggggcc ggtccggccg gagctctggc tgctgctgtg 300

ggcagccgcg tggcgcctgg gtgcctcggc gtgccccgcc ctctgcacct gcaccggaac 360

cacggtggac tgccacggca cggggctgca ggccattccc aagaatatac ctcggaacac 420

cgagcgcctg gaactcaatg gcaacaacat cactcggatc cataagaatg actttgcggg 480

gctcaagcag ctgcgggtgc tgcagctgat ggagaaccag attggagcag tggaacgtgg 540

tgcttttgat gacatgaagg agctggagcg gctgcgactg aaccgaaacc agctgcacat 600

gttaccggaa ctgctgttcc agaacaacca ggctttgtca agactgatct atggctcaga 660

gaggtgattt gccttggacc acagagcagc ttgacaatag ggttggaatt agacaccagg 720

ccttctaggg gccctgaaga gcctgaaaat ggtcttaaac cntgacc 767

<210> 6
<211> 1534
<212> PRT
<213> Homo sapiens
<400> 6

Met Ala Leu Thr Pro Gly Trp Gly Ser Ser Ala Gly Pro Val Arg Pro
1               5                   10                  15

Glu Leu Trp Leu Leu Leu Trp Ala Ala Ala Trp Arg Leu Gly Ala Ser
            20                  25                  30

Ala Cys Pro Ala Leu Cys Thr Cys Thr Gly Thr Thr Val Asp Cys His
        35                  40                  45

Gly Thr Gly Leu Gln Ala Ile Pro Lys Asn Ile Pro Arg Asn Thr Glu
    50                  55                  60

Arg Leu Glu Leu Asn Gly Asn Asn Ile Thr Arg Ile His Lys Asn Asp
65                  70                  75                  80

Phe Ala Gly Leu Lys Gln Leu Arg Val Leu Gln Leu Met Glu Asn Gln
                85                  90                  95

Ile Gly Ala Val Glu Arg Gly Ala Phe Asp Asp Met Lys Glu Leu Glu
            100                 105                 110

Arg Leu Arg Leu Asn Arg Asn Gln Leu His Met Leu Pro Glu Leu Leu
        115                 120                 125

Phe Gln Asn Asn Gln Ala Leu Ser Arg Leu Asp Leu Ser Glu Asn Ala
    130                 135                 140

Ile Gln Ala Ile Pro Arg Lys Ala Phe Arg Gly Ala Thr Asp Leu Lys
145                 150                 155                 160

Asn Leu Arg Leu Asp Lys Asn Gln Ile Ser Cys Ile Glu Glu Gly Ala
                165                 170                 175

Phe Arg Ala Leu Arg Gly Leu Glu Val Leu Thr Leu Asn Asn Asn Asn
            180                 185                 190

Ile Thr Thr Ile Pro Val Ser Ser Phe Asn His Met Pro Lys Leu Arg
        195                 200                 205

Thr Phe Arg Leu His Ser Asn His Leu Phe Cys Asp Cys His Leu Ala
    210                 215                 220

Trp Leu Ser Gln Trp Leu Arg Gln Arg Pro Thr Ile Gly Leu Phe Thr
225                 230                 235                 240

Gln Cys Ser Gly Pro Ala Ser Leu Arg Gly Leu Asn Val Ala Glu Val
                245                 250                 255

Gln Lys Ser Glu Phe Ser Cys Ser Gly Gln Gly Glu Ala Gly Arg Val
            260                 265                 270

Pro Thr Cys Thr Leu Ser Ser Gly Ser Cys Pro Ala Met Cys Thr Cys

275                 280                 285

Ser Asn Gly Ile Val Asp Cys Arg Gly Lys Gly Leu Thr Ala Ile Pro
    290                 295                 300

Ala Asn Leu Pro Glu Thr Met Thr Glu Ile Arg Leu Glu Leu Asn Gly
305                 310                 315                 320

Ile Lys Ser Ile Pro Pro Gly Ala Phe Ser Pro Tyr Arg Lys Leu Arg
                325                 330                 335

Arg Ile Asp Leu Ser Asn Asn Gln Ile Ala Glu Ile Ala Pro Asp Ala
            340                 345                 350

Phe Gln Gly Leu Arg Ser Leu Asn Ser Leu Val Leu Tyr Gly Asn Lys
        355                 360                 365

Ile Thr Asp Leu Pro Arg Gly Val Phe Gly Gly Leu Tyr Thr Leu Gln
    370                 375                 380

Leu Leu Leu Leu Asn Ala Asn Lys Ile Asn Cys Ile Arg Pro Asp Ala
385                 390                 395                 400

Phe Gln Asp Leu Gln Asn Leu Ser Leu Leu Ser Leu Tyr Asp Asn Lys
                405                 410                 415

Ile Gln Ser Leu Ala Lys Gly Thr Phe Thr Ser Leu Arg Ala Ile Gln
            420                 425                 430

Thr Leu His Leu Ala Gln Asn Pro Phe Ile Cys Asp Cys Asn Leu Lys
        435                 440                 445

Trp Leu Ala Asp Phe Leu Arg Thr Asn Pro Ile Glu Thr Ser Gly Ala
    450                 455                 460

Arg Cys Ala Ser Pro Arg Arg Leu Ala Asn Lys Arg Ile Gly Gln Ile
465                 470                 475                 480

Lys Ser Lys Lys Phe Arg Cys Ser Ala Lys Glu Gln Tyr Phe Ile Pro
                485                 490                 495

Gly Thr Glu Asp Tyr Gln Leu Asn Ser Glu Cys Asn Ser Asp Val Val
            500                 505                 510

Cys Pro His Lys Cys Arg Cys Glu Ala Asn Val Val Glu Cys Ser Ser
        515                 520                 525

Leu Lys Leu Thr Lys Ile Pro Glu Arg Ile Pro Gln Ser Thr Ala Glu
    530                 535                 540

Leu Arg Leu Asn Asn Asn Glu Ile Ser Ile Leu Glu Ala Thr Gly Met
545                 550                 555                 560

Phe Lys Lys Leu Thr His Leu Lys Lys Ile Asn Leu Ser Asn Asn Lys
                565                 570                 575

Val Ser Glu Ile Glu Asp Gly Ala Phe Glu Gly Ala Ala Ser Val Ser
            580                 585                 590

Glu Leu His Leu Thr Ala Asn Gln Leu Glu Ser Ile Arg Ser Gly Met
        595                 600                 605

Phe Arg Gly Leu Asp Gly Leu Arg Thr Leu Met Leu Arg Asn Asn Arg
    610                 615                 620

Ile Ser Cys Ile His Asn Asp Ser Phe Thr Gly Leu Arg Asn Val Arg
625                 630                 635                 640

Leu Leu Ser Leu Tyr Asp Asn Gln Ile Thr Thr Val Ser Pro Gly Ala
                645                 650                 655

Phe Asp Thr Leu Gln Ser Leu Ser Thr Leu Asn Leu Leu Ala Asn Pro
            660                 665                 670

Phe Asn Cys Asn Cys Gln Leu Ala Trp Leu Gly Gly Trp Leu Arg Lys
        675                 680                 685

Arg Lys Ile Val Thr Gly Asn Pro Arg Cys Gln Asn Pro Asp Phe Leu
    690                 695                 700

Arg Gln Ile Pro Leu Gln Asp Val Ala Phe Pro Asp Phe Arg Cys Glu
705                 710                 715                 720

Glu Gly Gln Glu Glu Gly Gly Cys Leu Pro Arg Pro Gln Cys Pro Gln
                725                 730                 735

Glu Cys Ala Cys Leu Asp Thr Val Val Arg Cys Ser Asn Lys His Leu
            740                 745                 750

Arg Ala Leu Pro Lys Gly Ile Pro Lys Asn Val Thr Glu Leu Tyr Leu
        755                 760                 765

Asp Gly Asn Gln Phe Thr Leu Val Pro Gly Gln Leu Ser Thr Phe Lys
    770                 775                 780

Tyr Leu Gln Leu Val Asp Leu Ser Asn Asn Lys Ile Ser Ser Leu Ser
785                 790                 795                 800

Asn Ser Ser Phe Thr Asn Met Ser Gln Leu Thr Thr Leu Ile Leu Ser
                805                 810                 815

Tyr Asn Ala Leu Gln Cys Ile Pro Pro Leu Ala Phe Gln Gly Leu Arg
            820                 825                 830

Ser Leu Arg Leu Leu Ser Leu His Gly Asn Asp Ile Ser Thr Leu Gln
        835                 840                 845

Glu Gly Ile Phe Ala Asp Val Thr Ser Leu Ser His Leu Ala Ile Gly
    850                 855                 860

Ala Asn Pro Leu Tyr Cys Asp Cys His Leu Arg Trp Leu Ser Ser Trp
865                 870                 875                 880

Val Lys Thr Gly Tyr Lys Glu Pro Gly Ile Ala Arg Cys Ala Gly Pro
                885                 890                 895

Gln Asp Met Glu Gly Lys Leu Leu Leu Thr Thr Pro Ala Lys Lys Phe
            900                 905                 910

Glu Cys Gln Gly Pro Pro Thr Leu Ala Val Gln Ala Lys Cys Asp Leu
        915                 920                 925

Cys Leu Ser Ser Pro Cys Gln Asn Gln Gly Thr Cys His Asn Asp Pro
    930                 935                 940

Leu Glu Val Tyr Arg Cys Ala Cys Pro Ser Gly Tyr Lys Gly Arg Asp
945                 950                 955                 960

Cys Glu Val Ser Leu Asn Ser Cys Ser Ser Gly Pro Cys Glu Asn Gly
                965                 970                 975

Gly Thr Cys His Ala Gln Glu Gly Glu Asp Ala Pro Phe Thr Cys Ser
            980                 985                 990

Cys Pro Thr Gly Phe Glu Gly Pro  Thr Cys Gly Val Asn  Thr Asp Asp
        995                 1000                 1005

Cys Val  Asp His Ala Cys Ala  Asn Gly Gly Val Cys  Val Asp Gly
    1010                 1015                 1020

Val Gly  Asn Tyr Thr Cys Gln  Cys Pro Leu Gln Tyr  Glu Gly Lys
    1025                 1030                 1035

Ala Cys Glu Gln Leu Val Asp Leu Cys Ser Pro Asp Leu Asn Pro
    1040                1045                1050

Cys Gln His Glu Ala Gln Cys Val Gly Thr Pro Asp Gly Pro Arg
    1055                1060                1065

Cys Glu Cys Met Pro Gly Tyr Ala Gly Asp Asn Cys Ser Glu Asn
    1070                1075                1080

Gln Asp Asp Cys Arg Asp His Arg Cys Gln Asn Gly Ala Gln Cys
    1085                1090                1095

Met Asp Glu Val Asn Ser Tyr Ser Cys Leu Cys Ala Glu Gly Tyr
    1100                1105                1110

Ser Gly Gln Leu Cys Glu Ile Pro Pro His Leu Pro Ala Pro Lys
    1115                1120                1125

Ser Pro Cys Glu Gly Thr Glu Cys Gln Asn Gly Ala Asn Cys Val
    1130                1135                1140

Asp Gln Gly Asn Arg Pro Val Cys Gln Cys Leu Pro Gly Phe Gly
    1145                1150                1155

Gly Pro Glu Cys Glu Lys Leu Leu Ser Val Asn Phe Val Asp Arg
    1160                1165                1170

Asp Thr Tyr Leu Gln Phe Thr Asp Leu Gln Asn Trp Pro Arg Ala
    1175                1180                1185

Asn Ile Thr Leu Gln Val Ser Thr Ala Glu Asp Asn Gly Ile Leu
    1190                1195                1200

Leu Tyr Asn Gly Asp Asn Asp His Ile Ala Val Glu Leu Tyr Gln
    1205                1210                1215

Gly His Val Arg Val Ser Tyr Asp Pro Gly Ser Tyr Pro Ser Ser
    1220                1225                1230

Ala Ile Tyr Ser Ala Glu Thr Ile Asn Asp Gly Gln Phe His Thr
    1235                1240                1245

Val Glu Leu Val Ala Phe Asp Gln Met Val Asn Leu Ser Ile Asp
    1250                1255                1260

Gly Gly Ser Pro Met Thr Met Asp Asn Phe Gly Lys His Tyr Thr

1265                 1270                 1275

Leu Asn  Ser Glu Ala Pro Leu  Tyr Val Gly Gly Met  Pro Val Asp
    1280                 1285                 1290

Val Asn  Ser Ala Ala Phe Arg  Leu Trp Gln Ile Leu  Asn Gly Thr
    1295                 1300                 1305

Gly Phe  His Gly Cys Ile Arg  Asn Leu Tyr Ile Asn  Asn Glu Leu
    1310                 1315                 1320

Gln Asp  Phe Thr Lys Thr Gln  Met Lys Pro Gly Val  Val Pro Gly
    1325                 1330                 1335

Cys Glu  Pro Cys Arg Lys Leu  Tyr Cys Leu His Gly  Ile Cys Gln
    1340                 1345                 1350

Pro Asn  Ala Thr Pro Gly Pro  Met Cys His Cys Glu  Ala Gly Trp
    1355                 1360                 1365

Val Gly  Leu His Cys Asp Gln  Pro Ala Asp Gly Pro  Cys His Gly
    1370                 1375                 1380

His Lys  Cys Val His Gly Gln  Cys Val Pro Leu Asp  Ala Leu Ser
    1385                 1390                 1395

Tyr Ser  Cys Gln Cys Gln Asp  Gly Tyr Ser Gly Ala  Leu Cys Asn
    1400                 1405                 1410

Gln Ala  Gly Ala Leu Ala Glu  Pro Cys Arg Gly Leu  Gln Cys Leu
    1415                 1420                 1425

His Gly  His Cys Gln Ala Ser  Gly Thr Lys Gly Ala  His Cys Val
    1430                 1435                 1440

Cys Asp  Pro Gly Phe Ser Gly  Glu Leu Cys Glu Gln  Glu Ser Glu
    1445                 1450                 1455

Cys Arg  Gly Asp Pro Val Arg  Asp Phe His Gln Val  Gln Arg Gly
    1460                 1465                 1470

Tyr Ala  Ile Cys Gln Thr Thr  Arg Pro Leu Ser Trp  Val Glu Cys
    1475                 1480                 1485

Arg Gly  Ser Cys Pro Gly Gln  Gly Cys Cys Gln Gly  Leu Arg Leu
    1490                 1495                 1500

Lys Arg Arg Lys Phe Thr Phe Glu Cys Ser Asp Gly Thr Ser Phe
    1505                1510                1515

Ala Glu Glu Val Glu Lys Pro Thr Lys Cys Gly Cys Ala Leu Cys
    1520                1525                1530

Ala

<210> 7
<211> 1461
<212> PRT
<213> Homo sapiens
<400> 7

Met Ala Leu Thr Pro Gly Trp Gly Ser Ser Ala Gly Pro Val Arg Pro
1               5                   10                  15

Glu Leu Trp Leu Leu Leu Trp Ala Ala Ala Trp Arg Leu Gly Ala Ser
            20                  25                  30

Ala Cys Pro Ala Leu Cys Thr Cys Thr Gly Thr Thr Val Asp Cys His
        35                  40                  45

Gly Thr Gly Leu Gln Ala Ile Pro Lys Asn Ile Pro Arg Asn Thr Glu
    50                  55                  60

Arg Leu Glu Leu Asn Gly Asn Asn Ile Thr Arg Ile His Lys Asn Asp
65                  70                  75                  80

Phe Ala Gly Leu Lys Gln Leu Arg Val Leu Gln Leu Met Glu Asn Gln
                85                  90                  95

Ile Gly Ala Val Glu Arg Gly Ala Phe Asp Asp Met Lys Glu Leu Glu
            100                 105                 110

Arg Leu Arg Leu Asn Arg Asn Gln Leu His Met Leu Pro Glu Leu Leu
        115                 120                 125

Phe Gln Asn Asn Gln Ala Leu Ser Arg Leu Asp Leu Ser Glu Asn Ala
    130                 135                 140

Ile Gln Ala Ile Pro Arg Lys Ala Phe Arg Gly Ala Thr Asp Leu Lys
145                 150                 155                 160

Asn Leu Arg Leu Asp Lys Asn Gln Ile Ser Cys Ile Glu Glu Gly Ala
                165                 170                 175

Phe Arg Ala Leu Arg Gly Leu Glu Val Leu Thr Leu Asn Asn Asn Asn

180                 185                 190

Ile Thr Thr Ile Pro Val Ser Ser Phe Asn His Met Pro Lys Leu Arg
        195                 200                 205

Thr Phe Arg Leu His Ser Asn His Leu Phe Cys Asp Cys His Leu Ala
    210                 215                 220

Trp Leu Ser Gln Trp Leu Arg Gln Arg Pro Thr Ile Gly Leu Phe Thr
225                 230                 235                 240

Gln Cys Ser Gly Pro Ala Ser Leu Arg Gly Leu Asn Val Ala Glu Val
                245                 250                 255

Gln Lys Ser Glu Phe Ser Cys Ser Gly Gln Gly Glu Ala Gly Arg Val
            260                 265                 270

Pro Thr Cys Thr Leu Ser Ser Gly Ser Cys Pro Ala Met Cys Thr Cys
        275                 280                 285

Ser Asn Gly Ile Val Asp Cys Arg Gly Lys Gly Leu Thr Ala Ile Pro
    290                 295                 300

Ala Asn Leu Pro Glu Thr Met Thr Glu Ile Arg Leu Glu Leu Asn Gly
305                 310                 315                 320

Ile Lys Ser Ile Pro Pro Gly Ala Phe Ser Pro Tyr Arg Lys Leu Arg
                325                 330                 335

Arg Ile Asp Leu Ser Asn Asn Gln Ile Ala Glu Ile Ala Pro Asp Ala
            340                 345                 350

Phe Gln Gly Leu Arg Ser Leu Asn Ser Leu Val Leu Tyr Gly Asn Lys
        355                 360                 365

Ile Thr Asp Leu Pro Arg Gly Val Phe Gly Gly Leu Tyr Thr Leu Gln
    370                 375                 380

Leu Leu Leu Leu Asn Ala Asn Lys Ile Asn Cys Ile Arg Pro Asp Ala
385                 390                 395                 400

Phe Gln Asp Leu Gln Asn Leu Ser Leu Leu Ser Leu Tyr Asp Asn Lys
                405                 410                 415

Ile Gln Ser Leu Ala Lys Gly Thr Phe Thr Ser Leu Arg Ala Ile Gln
            420                 425                 430

Thr Leu His Leu Ala Gln Asn Pro Phe Ile Cys Asp Cys Asn Leu Lys
        435                 440                 445

Trp Leu Ala Asp Phe Leu Arg Thr Asn Pro Ile Glu Thr Ser Gly Ala
    450                 455                 460

Arg Cys Ala Ser Pro Arg Arg Leu Ala Asn Lys Arg Ile Gly Gln Ile
465                 470                 475                 480

Lys Ser Lys Lys Phe Arg Cys Ser Ala Lys Glu Gln Tyr Phe Ile Pro
                485                 490                 495

Gly Thr Glu Asp Tyr Gln Leu Asn Ser Glu Cys Asn Ser Asp Val Val
            500                 505                 510

Cys Pro His Lys Cys Arg Cys Glu Ala Asn Val Val Glu Cys Ser Ser
        515                 520                 525

Leu Lys Leu Thr Lys Ile Pro Glu Arg Ile Pro Gln Ser Thr Ala Glu
    530                 535                 540

Leu Arg Leu Asn Asn Asn Glu Ile Ser Ile Leu Glu Ala Thr Gly Met
545                 550                 555                 560

Phe Lys Lys Leu Thr His Leu Lys Lys Ile Asn Leu Ser Asn Asn Lys
                565                 570                 575

Val Ser Glu Ile Glu Asp Gly Ala Phe Glu Gly Ala Ala Ser Val Ser
            580                 585                 590

Glu Leu His Leu Thr Ala Asn Gln Leu Glu Ser Ile Arg Ser Gly Met
        595                 600                 605

Phe Arg Gly Leu Asp Gly Leu Arg Thr Leu Met Leu Arg Asn Asn Arg
    610                 615                 620

Ile Ser Cys Ile His Asn Asp Ser Phe Thr Gly Leu Arg Asn Val Arg
625                 630                 635                 640

Leu Leu Ser Leu Tyr Asp Asn Gln Ile Thr Thr Val Ser Pro Gly Ala
                645                 650                 655

Phe Asp Thr Leu Gln Ser Leu Ser Thr Leu Asn Leu Leu Ala Asn Pro
            660                 665                 670

Phe Asn Cys Asn Cys Gln Leu Ala Trp Leu Gly Gly Trp Leu Arg Lys
        675                 680                 685

Arg Lys Ile Val Thr Gly Asn Pro Arg Cys Gln Asn Pro Asp Phe Leu
    690                 695                 700

Arg Gln Ile Pro Leu Gln Asp Val Ala Phe Pro Asp Phe Arg Cys Glu
705                 710                 715                 720

Glu Gly Gln Glu Glu Gly Gly Cys Leu Pro Arg Pro Gln Cys Pro Gln
                725                 730                 735

Glu Cys Ala Cys Leu Asp Thr Val Val Arg Cys Ser Asn Lys His Leu
            740                 745                 750

Arg Ala Leu Pro Lys Gly Ile Pro Lys Asn Val Thr Glu Leu Tyr Leu
        755                 760                 765

Asp Gly Asn Gln Phe Thr Leu Val Pro Gly Gln Leu Ser Thr Phe Lys
    770                 775                 780

Tyr Leu Gln Leu Val Asp Leu Ser Asn Asn Lys Ile Ser Ser Leu Ser
785                 790                 795                 800

Asn Ser Ser Phe Thr Asn Met Ser Gln Leu Thr Thr Leu Ile Leu Ser
                805                 810                 815

Tyr Asn Ala Leu Gln Cys Ile Pro Pro Leu Ala Phe Gln Gly Leu Arg
            820                 825                 830

Ser Leu Arg Leu Leu Ser Leu His Gly Asn Asp Ile Ser Thr Leu Gln
        835                 840                 845

Glu Gly Ile Phe Ala Asp Val Thr Ser Leu Ser His Leu Ala Ile Gly
    850                 855                 860

Ala Asn Pro Leu Tyr Cys Asp Cys His Leu Arg Trp Leu Ser Ser Trp
865                 870                 875                 880

Val Lys Thr Gly Tyr Lys Glu Pro Gly Ile Ala Arg Cys Ala Gly Pro
                885                 890                 895

Gln Asp Met Glu Gly Lys Leu Leu Leu Thr Thr Pro Ala Lys Lys Phe
            900                 905                 910

Glu Cys Gln Gly Pro Pro Thr Leu Ala Val Gln Ala Lys Cys Asp Leu
        915                 920                 925

Cys Leu Ser Ser Pro Cys Gln Asn Gln Gly Thr Cys His Asn Asp Pro
    930                 935                 940

Leu Glu Val Tyr Arg Cys Ala Cys Pro Ser Gly Tyr Lys Gly Arg Asp
945                 950                 955                 960

Cys Glu Val Ser Leu Asn Ser Cys Ser Ser Gly Pro Cys Glu Asn Gly
                965                 970                 975

Gly Thr Cys His Ala Gln Glu Gly Glu Asp Ala Pro Phe Thr Cys Ser
            980                 985                 990

Cys Pro Thr Gly Phe Glu Gly Pro  Thr Cys Gly Val Asn  Thr Asp Asp
        995                 1000                 1005

Cys Val  Asp His Ala Cys Ala  Asn Gly Gly Val Cys  Val Asp Gly
    1010                 1015                 1020

Val Gly  Asn Tyr Thr Cys Gln  Cys Pro Leu Gln Tyr  Glu Gly Lys
    1025                 1030                 1035

Ala Cys  Glu Gln Leu Val Asp  Leu Cys Ser Pro Asp  Leu Asn Pro
    1040                 1045                 1050

Cys Gln  His Glu Ala Gln Cys  Val Gly Thr Pro Asp  Gly Pro Arg
    1055                 1060                 1065

Cys Glu  Cys Met Pro Gly Tyr  Ala Gly Asp Asn Cys  Ser Glu Asn
    1070                 1075                 1080

Gln Asp  Asp Cys Arg Asp His  Arg Cys Gln Asn Gly  Ala Gln Cys
    1085                 1090                 1095

Met Asp  Glu Val Asn Ser Tyr  Ser Cys Leu Cys Ala  Glu Gly Tyr
    1100                 1105                 1110

Ser Gly  Gln Leu Cys Glu Ile  Pro Pro His Leu Pro  Ala Pro Lys
    1115                 1120                 1125

Ser Pro  Cys Glu Gly Thr Glu  Cys Gln Asn Gly Ala  Asn Cys Val
    1130                 1135                 1140

Asp Gln  Gly Asn Arg Pro Val  Cys Gln Cys Leu Pro  Gly Phe Gly
    1145                 1150                 1155

Gly Pro  Glu Cys Glu Lys Leu  Leu Ser Val Asn Phe  Val Asp Arg
    1160                 1165                 1170

Asp Thr  Tyr Leu Gln Phe Thr  Asp Leu Gln Asn Trp  Pro Arg Ala

1175                 1180                 1185

Asn Ile  Thr Leu Gln Val Ser  Thr Ala Glu Asp Asn  Gly Ile Leu
    1190                 1195                 1200

Leu Tyr  Asn Gly Asp Asn Asp  His Ile Ala Val Glu  Leu Tyr Gln
    1205                 1210                 1215

Gly His  Val Arg Val Ser Tyr  Asp Pro Gly Ser Tyr  Pro Ser Ser
    1220                 1225                 1230

Ala Ile  Tyr Ser Ala Glu Thr  Ile Asn Asp Gly Gln  Phe His Thr
    1235                 1240                 1245

Val Glu  Leu Val Ala Phe Asp  Gln Met Val Asn Leu  Ser Ile Asp
    1250                 1255                 1260

Gly Gly  Ser Pro Met Thr Met  Asp Asn Phe Gly Lys  His Tyr Thr
    1265                 1270                 1275

Leu Asn  Ser Glu Ala Pro Leu  Tyr Val Gly Gly Met  Pro Val Asp
    1280                 1285                 1290

Val Asn  Ser Ala Ala Phe Arg  Leu Trp Gln Ile Leu  Asn Gly Thr
    1295                 1300                 1305

Gly Phe  His Gly Cys Ile Arg  Asn Leu Tyr Ile Asn  Asn Glu Leu
    1310                 1315                 1320

Gln Asp  Phe Thr Lys Thr Gln  Met Lys Pro Gly Val  Val Pro Gly
    1325                 1330                 1335

Cys Glu  Pro Cys Arg Lys Leu  Tyr Cys Leu His Gly  Ile Cys Gln
    1340                 1345                 1350

Pro Asn  Ala Thr Pro Gly Pro  Met Cys His Cys Glu  Ala Gly Trp
    1355                 1360                 1365

Val Gly  Leu His Cys Asp Gln  Pro Ala Asp Gly Pro  Cys His Gly
    1370                 1375                 1380

His Lys  Cys Val His Gly Gln  Cys Val Pro Leu Asp  Ala Leu Ser
    1385                 1390                 1395

Tyr Ser  Cys Gln Cys Gln Asp  Gly Tyr Ser Gly Ala  Leu Cys Asn
    1400                 1405                 1410

Gln Ala Gly Ala Leu Ala Glu Pro Cys Arg Gly Leu Gln Cys Leu
    1415                1420                1425

His Gly His Cys Gln Ala Ser Gly Thr Lys Gly Ala His Cys Val
    1430                1435                1440

Cys Asp Pro Gly Phe Ser Gly Glu Leu Cys Glu Gln Gly Gln Gly
    1445                1450                1455

Pro Pro Ser
    1460

<210> 8
<211> 144
<212> PRT
<213> Homo sapiens
<400> 8

Met Ala Leu Thr Pro Gly Trp Gly Ser Ser Ala Gly Pro Val Arg Pro
1               5                   10                  15

Glu Leu Trp Leu Leu Leu Trp Ala Ala Ala Trp Arg Leu Gly Ala Ser
            20                  25                  30

Ala Cys Pro Ala Leu Cys Thr Cys Thr Gly Thr Thr Val Asp Cys His
        35                  40                  45

Gly Thr Gly Leu Gln Ala Ile Pro Lys Asn Ile Pro Arg Asn Thr Glu
    50                  55                  60

Arg Leu Glu Leu Asn Gly Asn Asn Ile Thr Arg Ile His Lys Asn Asp
65                  70                  75                  80

Phe Ala Gly Leu Lys Gln Leu Arg Val Leu Gln Leu Met Glu Asn Gln
                85                  90                  95

Ile Gly Ala Val Glu Arg Gly Ala Phe Asp Asp Met Lys Glu Leu Glu
            100                 105                 110

Arg Leu Arg Leu Asn Arg Asn Gln Leu His Met Leu Pro Glu Leu Leu
        115                 120                 125

Phe Gln Asn Asn Gln Ala Leu Ser Arg Leu Ile Tyr Gly Ser Glu Arg
    130                 135                 140

<210> 9
<211> 16
<212> PRT
<213> Homo sapiens
<400> 9

Cys Ser Asp Gly Thr Ser Phe Ala Glu Glu Val Glu Lys Pro Thr Lys
1               5                   10                  15

<210> 10
<211> 16
<212> PRT
<213> Homo sapiens
<400> 10

Gln Tyr Phe Ile Pro Gly Thr Glu Asp Tyr Gln Leu Asn Ser Glu Cys
1               5                   10                  15

<210> 11
<211> 15
<212> PRT
<213> Homo sapiens
<400> 11

Gln Glu Ser Glu Cys Arg Gly Asp Pro Val Arg Asp Phe His Gln
1               5                   10                  15

<210> 12
<211> 14
<212> PRT
<213> Homo sapiens
<400> 12

Glu Glu Gly Gln Glu Glu Gly Gly Cys Leu Pro Arg Gln Cys
1               5                   10

<210> 13
<211> 15
<212> PRT
<213> Homo sapiens
<400> 13

Asn Asn Glu Leu Gln Asp Phe Thr Lys Thr Gln Met Lys Pro Gly
1               5                   10                  15

<210> 14
<211> 8
<212> PRT
<213> Homo sapiens
<400> 14

Cys Glu Gln Gly Gln Pro Pro Ser
1               5

<210> 15
<211> 22
<212> DNA
<213> Artificial
<220>
<221> artificial
<222> (1)..(22)
<223> antisense

<400>  15
ggggauggug gugauauugu ug                                              22

<210>  16
<211>  21
<212>  DNA
<213>  Artificial
<220>
<221>  artificial
<222>  (1)..(21)
<223>  antisense
<400>  16
agauggggag ggaucucaca g                                               21

<210>  17
<211>  21
<212>  DNA
<213>  Artificial
<220>
<221>  artificial
<222>  (1)..(21)
<223>  antisense
<400>  17
ugaacuuccu ccgcuucagc c                                               21

<210>  18
<211>  22
<212>  DNA
<213>  Artificial
<220>
<221>  artificial
<222>  (1)..(22)
<223>  antisense
<400>  18
   uuucuccagc uucucccugc cc                                               22

<210>  19
<211>  22
<212>  DNA
<213>  Artificial
<220>
<221>  artificial
<222>  (1)..(22)
<223>  antisense
<400>  19
cuauugucaa gcugcucugu gg                                              22

<210>  20
<211>  26
<212>  DNA
<213>  Artificial
<220>
<221>  artificial
<222>  (1)..(26)
<223>  ribozyme
<400>  20
guuccugaug agucgacacg aaacug                                          26

<210> 21
<211> 26
<212> DNA
<213> Artificial
<220>
<221> artificial
<222> (1)..(26)
<223> ribozyme
<400> 21
caggcugaug agucgacacg aaagau 26

<210> 22
<211> 26
<212> DNA
<213> Artificial
<220>
<221> artificial
<222> (1)..(26)
<223> ribozyme
<400> 22
ccggcugaug agucgacacg aaacuc 26

<210> 23
<211> 26
<212> DNA
<213> Artificial
<220>
<221> artificial
<222> (1)..(26)
<223> ribozyme
<400> 23
gauucugaug agucgacacg aaaugc 26

<210> 24
<211> 26
<212> DNA
<213> Artificial
<220>
<221> artificial
<222> (1)..(26)
<223> ribozyme
<400> 24
ucuucugaug agucgacacg aaaggg 26

<210> 25
<211> 3386
<212> DNA
<213> Homo sapiens
<400> 25
gagggcacca tggcgctgac tcccgggtgg gggtcctcgg cggggccggt ccggccggag 60

ctctggctgc tgctgtgggc agccgcgtgg cgcctgggtg cctcggcgtg ccccgccctc 120

tgcacctgca ccggaaccac ggtggactgc cacggcacgg ggctgcaggc cattcccaag 180

aatatacctc ggaacaccga gcgcctggaa ctcaatggca acaacatcac tcggatccat 240

aagaatgact ttgcggggct caagcagctg cgggtgctgc agctgatgga gaaccagatt 300

ggagcagtgg aacgtggtgc ttttgatgac atgaaggagc tggagcggct gcgactgaac 360
cgaaaccagc tgcacatgtt accggaactg ctgttccaga acaaccaggc tttgtcaaga 420
ctggacttga gtgagaacgc catccaggcc atccccagga aagcttttcg gggagctacg 480
gaccttaaaa atttacagct ggacaagaac cagatcagct gcattgagga aggggccttc 540
cgtgctctgc gggggctgga ggtgctgacc ctgaacaaca acaatatcac caccatcccc 600
gtgtccagct tcaaccatat gcccaagcta cggaccttcc gcctgcactc caaccacctg 660
ttttgcgact gccacctggc ctggctctcg cagtggctga ggcagcggcc aaccatcggg 720
ctcttcaccc agtgctcggg cccagccagc ctgcgtggcc tcaatgtggc agaggtccag 780
aagagtgagt tcagctgctc aggccaggga gaagcggggc gcgtgcccac ctgcaccctg 840
tcctccggct cctgcccggc catgtgcacc tgcagcaatg gcatcgtgga ctgtcgtgga 900
aaaggcctca ctgccatccc ggccaacctg cccgagacca tgacggagat acgcctggag 960
ctgaacggca tcaagtccat ccctcctgga gccttctcac cctacagaaa gctacggagg 1020
atagacctga gcaacaatca gatcgctgag attgcacccg acgccttcca gggcctccgc 1080
tccctgaact cgctggtcct ctatggaaac aagatcacag acctcccccg tggtgtgttt 1140
ggaggcctat acaccctaca gctcctgctc ctgaatgcca acaagatcaa ctgcatccgg 1200
cccgatgcct tccaggacct gcagaacctc tcactgctct ccctgtatga caacaagatc 1260
cagagcctcg ccaagggcac tttcacctcc ctgcgggcca tccagactct gcacctggcg 1320
cagaaccctt tcatttgcga ctgtaacctc aagtggctgg cagacttcct gcgcaccaat 1380
cccatcgaga cgagtggtgc ccgctgtgcc agtccccggc gcctcgccaa caagcgcatc 1440
gggcagatca agagcaagaa gttccggtgc tcagccaaag agcagtactt cattccaggc 1500
acggaggatt accagctgaa cagcgagtgc aacagcgacg tggtctgtcc ccacaagtgc 1560
cgctgtgagg ccaacgtggt ggagtgctcc agcctgaagc tcaccaagat ccctgagcgc 1620
atcccccagt ccacggcaga actgcgattg aataacaatg agatttccat cctggaggcc 1680
actgggatgt ttaaaaaact tacacatctg aagaaaatca atctgagcaa caacaaggtg 1740
tcagaaattg aagatggggc cttcgagggc gcagcctctg tgagcgagct gcacctaact 1800
gccaaccagc tggagtccat ccggagcggc atgttccggg gtctggatgg cttgaggacc 1860
ctaatgctgc ggaacaaccg catcagctgc atccacaacg acagcttcac gggcctgcgc 1920
aacgtccggc tcctctcgct ctacgacaac cagatcacca ccgtatcccc aggagccttc 1980
gacaccctcc agtccctctc cacactgaat ctcctggcca accctttcaa ctgcaactgc 2040
cagctggcct ggctaggagg ctggctacgg aagcgcaaga tcgtgacggg gaacccgcga 2100
tgccagaacc ctgacttttt gcggcagatt cccctgcagg acgtggcctt ccctgacttc 2160

aggtgtgagg aaggccagga ggaggggggc tgcctgcccc gcccacagtg cccacaggag 2220

tgcgcctgcc tggacaccgt ggtccgatgc agcaacaagc acctgcgggc cctgcccaag 2280

ggcattccca agaatgtcac agaactctat ttggacggga accagttcac gctggttccg 2340

ggacagctgt ctaccttcaa gtacctgcag ctcgtggacc tgagcaacaa caagatcagt 2400

tccttaagca attcctcctt caccaacatg agccagctga ccactctgat cctcagctac 2460

aatgccctgc agtgcatcct gcctttggcc ttccagggac tccgctccct gcgcctgctg 2520

tctctccacg gcaatgacat ctccaccctc caagagggca tctttgcaga cgtgacctcc 2580

ctgtctcacc tggccattgg tgccaacccc ctatactgtg actgccacct ccgctggctg 2640

tccagctggg tgaagactgg ctacaaggaa ccgggcattg ctcgttgtgc tgggccccag 2700

gacatggagg gcaagctgct cctcaccacg cctgccaaga agtttgaatg ccaaggtcct 2760

ccaacgctgg ctgtccaggc caagtgtgat ctctgcttgt ccagtccgtg ccagaaccag 2820

ggcacctgcc acaacgaccc ccttgaggtg tacaggtgcg cctgccccag cggctataag 2880

ggtcgagact gtgaggtgtc cctggacagc tgttccagtg gcccctgtga aaatgggggc 2940

acctgccatg cacaggaggg cgaggatgcc ccgttcacgt gctcctgtcc caccggcttt 3000

gaaggaccaa cctgtggggt gaacacagat gactgtgtgg atcatgcctg tgccaatggg 3060

ggcgtctgtg tggatggtgt gggcaactac acctgccagt gccccctgca gtatgaggga 3120

aaggcctgtg agcagctggt ggacttgtgc tctccggatc tgaacccatg tcaacacgag 3180

gcccagtgtg tgggcacccc ggatgggccc aggtgtgagt gcatgccagg ttatgcaggt 3240

gacaactgca gtgagaacca ggatgactgc agggaccacc gctgccagaa tggggcccag 3300

tgtatggatg aagtcaacag ctactcctgc ctctgtgctg agggctacag tggacagctc 3360

tgtgagatcc ctccctaatt aggtga 3386

<210> 26
<211> 2804
<212> DNA
<213> Homo sapiens
<400> 26
gagggcacca tggcgctgac tcccgggtgg gggtcctcgg cggggccggt ccggccggag 60

ctctggctgc tgctgtgggc agccgcgtgg cgcctgggtg cctcggcgtg ccccgccctc 120

tgcacctgca ccggaaccac ggtggactgc cacggcacgg ggctgcaggc cattcccaag 180

aatatacctc ggaacaccga gcgcctggaa ctcaatggca acaacatcac tcggatccat 240

aagaatgact ttgcggggct caagcagctg cgggtgctgc agctgatgga gaaccagatt 300

ggagcagtgg aacgtggtgc ttttgatgac atgaaggagc tggagcggct gcgactgaac 360

cgaaaccagc tgcacatgtt accggaactg ctgttccaga acaaccaggc tttgtcaaga 420

ctggacttga gtgagaacgc catccaggcc atccccagga aagcttttcg gggagctacg 480

gaccttaaaa atttacagct ggacaagaac cagatcagct gcattgagga aggggccttc 540
cgtgctctgc gggggctgga ggtgctgacc ctgaacaaca acaatatcac caccatcccc 600
gtgtccagct tcaaccatat gcccaagcta cggaccttcc gcctgcactc caaccacctg 660
ttttgcgact gccacctggc ctggctctcg cagtggctga ggcagcggcc aaccatcggg 720
ctcttcaccc agtgctcggg cccagccagc ctgcgtggcc tcaatgtggc agaggtccag 780
aagagtgagt tcagctgctc aggccaggga gaagcggggc gcgtgcccac ctgcaccctg 840
tcctccggct cctgcccggc catgtgcacc tgcagcaatg gcatcgtgga ctgtcgtgga 900
aaaggcctca ctgccatccc ggccaacctg cccgagacca tgacggagat acgcctggag 960
ctgaacggca tcaagtccat ccctcctgga gccttctcac cctacagaaa gctacggagg 1020
atagacctga gcaacaatca gatcgctgag attgcacccg acgccttcca gggcctccgc 1080
tccctgaact cgctggtcct ctatggaaac aagatcacag acctcccccg tggtgtgttt 1140
ggaggcctat acaccctaca gctcctgctc ctgaatgcca acaagatcaa ctgcatccgg 1200
cccgatgcct tccaggacct gcagaacctc tcactgctct ccctgtatga caacaagatc 1260
cagagcctcg ccaagggcac tttcacctcc ctgcgggcca tccagactct gcacctggcg 1320
cagaaccctt tcatttgcga ctgtaacctc aagtggctgg cagacttcct gcgcaccaat 1380
cccatcgaga cgagtggtgc ccgctgtgcc agtccccggc gcctcgccaa caagcgcatc 1440
gggcagatca agagcaagaa gttccggtgc tcagccaaag agcagtactt cattccaggc 1500
acggaggatt accagctgaa cagcgagtgc aacagcgacg tggtctgtcc ccacaagtgc 1560
cgctgtgagg ccaacgtggt ggagtgctcc agcctgaagc tcaccaagat ccctgagcgc 1620
atcccccagt ccacggcaga actgcgattg aataacaatg agatttccat cctggaggcc 1680
actgggatgt ttaaaaaact tacacatctg aagaaaatca atctgagcaa caacaaggtg 1740
tcagaaattg aagatggggc cttcgagggc gcagcctctg tgagcgagct gcacctaact 1800
gccaaccagc tggagtccat ccggagcggc atgttccggg gtctggatgg cttgaggacc 1860
ctaatgctgc ggaacaaccg catcagctgc atccacaacg acagcttcac gggcctgcgc 1920
aacgtccggc tcctctcgct ctacgacaac cagatcacca ccgtatcccc aggagccttc 1980
gacaccctcc agtccctctc cacactgaat ctcctggcca accctttcaa ctgcaactgc 2040
cagctggcct ggctaggagg ctggctacgg aagcgcaaga tcgtgacggg gaacccgcga 2100
tgccagaacc ctgacttttt gcggcagatt cccctgcagg acgtggcctt ccctgacttc 2160
aggtgtgagg aaggccagga ggaggggggc tgcctgcccc gcccacagtg cccacaggag 2220
tgcgcctgcc tggacaccgt ggtccgatgc agcaacaagc acctgcgggc cctgcccaag 2280
ggcattccca agaatgtcac agaactctat ttggacggga accagttcac gctggttccg 2340

ggacagctgt ctaccttcaa gtacctgcag ctcgtggacc tgagcaacaa caagatcagt 2400

tccttaagca attcctcctt caccaacatg agccagctga ccactctgat cctcagctac 2460

aatgccctgc agtgcatcct gcctttggcc ttccagggac tccgctccct gcgcctgctg 2520

tctctccacg gcaatgacat ctccaccctc caagagggca tctttgcaga cgtgacctcc 2580

ctgtctcacc tggccattgg tgccaacccc ctatactgtg actgccacct ccgctggctg 2640

tccagctggg tgaagactgg ctacaaggaa ccgggcattg ctcgttgtgc tgggccccag 2700

gacatggagg gcaagctgct cctcaccacg cctgccaaga agtttgaatg ccaaggtcct 2760

ccaacgctgg ctgtccaggc caagtgtgat ctctaaatga atag 2804

<210> 27
<211> 1122
<212> PRT
<213> Homo sapiens
<400> 27

Met Ala Leu Thr Pro Gly Trp Gly Ser Ser Ala Gly Pro Val Arg Pro
1               5                   10                  15

Glu Leu Trp Leu Leu Leu Trp Ala Ala Ala Trp Arg Leu Gly Ala Ser
            20                  25                  30

Ala Cys Pro Ala Leu Cys Thr Cys Thr Gly Thr Thr Val Asp Cys His
        35                  40                  45

Gly Thr Gly Leu Gln Ala Ile Pro Lys Asn Ile Pro Arg Asn Thr Glu
    50                  55                  60

Arg Leu Glu Leu Asn Gly Asn Asn Ile Thr Arg Ile His Lys Asn Asp
65                  70                  75                  80

Phe Ala Gly Leu Lys Gln Leu Arg Val Leu Gln Leu Met Glu Asn Gln
                85                  90                  95

Ile Gly Ala Val Glu Arg Gly Ala Phe Asp Asp Met Lys Glu Leu Glu
            100                 105                 110

Arg Leu Arg Leu Asn Arg Asn Gln Leu His Met Leu Pro Glu Leu Leu
        115                 120                 125

Phe Gln Asn Asn Gln Ala Leu Ser Arg Leu Asp Leu Ser Glu Asn Ala
    130                 135                 140

Ile Gln Ala Ile Pro Arg Lys Ala Phe Arg Gly Ala Thr Asp Leu Lys
145                 150                 155                 160

Asn Leu Gln Leu Asp Lys Asn Gln Ile Ser Cys Ile Glu Glu Gly Ala
                165                 170                 175

Phe Arg Ala Leu Arg Gly Leu Glu Val Leu Thr Leu Asn Asn Asn Asn
            180                 185                 190

Ile Thr Thr Ile Pro Val Ser Ser Phe Asn His Met Pro Lys Leu Arg
        195                 200                 205

Thr Phe Arg Leu His Ser Asn His Leu Phe Cys Asp Cys His Leu Ala
    210                 215                 220

Trp Leu Ser Gln Trp Leu Arg Gln Arg Pro Thr Ile Gly Leu Phe Thr
225                 230                 235                 240

Gln Cys Ser Gly Pro Ala Ser Leu Arg Gly Leu Asn Val Ala Glu Val
                245                 250                 255

Gln Lys Ser Glu Phe Ser Cys Ser Gly Gln Gly Glu Ala Gly Arg Val
            260                 265                 270

Pro Thr Cys Thr Leu Ser Ser Gly Ser Cys Pro Ala Met Cys Thr Cys
        275                 280                 285

Ser Asn Gly Ile Val Asp Cys Arg Gly Lys Gly Leu Thr Ala Ile Pro
    290                 295                 300

Ala Asn Leu Pro Glu Thr Met Thr Glu Ile Arg Leu Glu Leu Asn Gly
305                 310                 315                 320

Ile Lys Ser Ile Pro Pro Gly Ala Phe Ser Pro Tyr Arg Lys Leu Arg
                325                 330                 335

Arg Ile Asp Leu Ser Asn Asn Gln Ile Ala Glu Ile Ala Pro Asp Ala
            340                 345                 350

Phe Gln Gly Leu Arg Ser Leu Asn Ser Leu Val Leu Tyr Gly Asn Lys
        355                 360                 365

Ile Thr Asp Leu Pro Arg Gly Val Phe Gly Gly Leu Tyr Thr Leu Gln
    370                 375                 380

Leu Leu Leu Leu Asn Ala Asn Lys Ile Asn Cys Ile Arg Pro Asp Ala
385                 390                 395                 400

Phe Gln Asp Leu Gln Asn Leu Ser Leu Leu Ser Leu Tyr Asp Asn Lys
                405                 410                 415

Ile Gln Ser Leu Ala Lys Gly Thr Phe Thr Ser Leu Arg Ala Ile Gln
            420                 425                 430

Thr Leu His Leu Ala Gln Asn Pro Phe Ile Cys Asp Cys Asn Leu Lys
        435                 440                 445

Trp Leu Ala Asp Phe Leu Arg Thr Asn Pro Ile Glu Thr Ser Gly Ala
    450                 455                 460

Arg Cys Ala Ser Pro Arg Arg Leu Ala Asn Lys Arg Ile Gly Gln Ile
465                 470                 475                 480

Lys Ser Lys Lys Phe Arg Cys Ser Ala Lys Glu Gln Tyr Phe Ile Pro
                485                 490                 495

Gly Thr Glu Asp Tyr Gln Leu Asn Ser Glu Cys Asn Ser Asp Val Val
            500                 505                 510

Cys Pro His Lys Cys Arg Cys Glu Ala Asn Val Val Glu Cys Ser Ser
        515                 520                 525

Leu Lys Leu Thr Lys Ile Pro Glu Arg Ile Pro Gln Ser Thr Ala Glu
    530                 535                 540

Leu Arg Leu Asn Asn Asn Glu Ile Ser Ile Leu Glu Ala Thr Gly Met
545                 550                 555                 560

Phe Lys Lys Leu Thr His Leu Lys Lys Ile Asn Leu Ser Asn Asn Lys
                565                 570                 575

Val Ser Glu Ile Glu Asp Gly Ala Phe Glu Gly Ala Ala Ser Val Ser
            580                 585                 590

Glu Leu His Leu Thr Ala Asn Gln Leu Glu Ser Ile Arg Ser Gly Met
        595                 600                 605

Phe Arg Gly Leu Asp Gly Leu Arg Thr Leu Met Leu Arg Asn Asn Arg
    610                 615                 620

Ile Ser Cys Ile His Asn Asp Ser Phe Thr Gly Leu Arg Asn Val Arg
625                 630                 635                 640

Leu Leu Ser Leu Tyr Asp Asn Gln Ile Thr Thr Val Ser Pro Gly Ala
                645                 650                 655

Phe Asp Thr Leu Gln Ser Leu Ser Thr Leu Asn Leu Leu Ala Asn Pro
            660                 665                 670

Phe Asn Cys Asn Cys Gln Leu Ala Trp Leu Gly Gly Trp Leu Arg Lys
        675                 680                 685

Arg Lys Ile Val Thr Gly Asn Pro Arg Cys Gln Asn Pro Asp Phe Leu
    690                 695                 700

Arg Gln Ile Pro Leu Gln Asp Val Ala Phe Pro Asp Phe Arg Cys Glu
705                 710                 715                 720

Glu Gly Gln Glu Glu Gly Gly Cys Leu Pro Arg Pro Gln Cys Pro Gln
                725                 730                 735

Glu Cys Ala Cys Leu Asp Thr Val Val Arg Cys Ser Asn Lys His Leu
            740                 745                 750

Arg Ala Leu Pro Lys Gly Ile Pro Lys Asn Val Thr Glu Leu Tyr Leu
        755                 760                 765

Asp Gly Asn Gln Phe Thr Leu Val Pro Gly Gln Leu Ser Thr Phe Lys
    770                 775                 780

Tyr Leu Gln Leu Val Asp Leu Ser Asn Asn Lys Ile Ser Ser Leu Ser
785                 790                 795                 800

Asn Ser Ser Phe Thr Asn Met Ser Gln Leu Thr Thr Leu Ile Leu Ser
                805                 810                 815

Tyr Asn Ala Leu Gln Cys Ile Leu Pro Leu Ala Phe Gln Gly Leu Arg
            820                 825                 830

Ser Leu Arg Leu Leu Ser Leu His Gly Asn Asp Ile Ser Thr Leu Gln
        835                 840                 845

Glu Gly Ile Phe Ala Asp Val Thr Ser Leu Ser His Leu Ala Ile Gly
    850                 855                 860

Ala Asn Pro Leu Tyr Cys Asp Cys His Leu Arg Trp Leu Ser Ser Trp
865                 870                 875                 880

Val Lys Thr Gly Tyr Lys Glu Pro Gly Ile Ala Arg Cys Ala Gly Pro
                885                 890                 895

Gln Asp Met Glu Gly Lys Leu Leu Leu Thr Thr Pro Ala Lys Lys Phe
            900                 905                 910

Glu Cys Gln Gly Pro Pro Thr Leu Ala Val Gln Ala Lys Cys Asp Leu

        915                 920                 925

Cys Leu Ser Ser Pro Cys Gln Asn Gln Gly Thr Cys His Asn Asp Pro
930 ·935 940

Leu Glu Val Tyr Arg Cys Ala Cys Pro Ser Gly Tyr Lys Gly Arg Asp
945 950 955 960

Cys Glu Val Ser Leu Asp Ser Cys Ser Ser Gly Pro Cys Glu Asn Gly
965 970 975

Gly Thr Cys His Ala Gln Glu Gly Glu Asp Ala Pro Phe Thr Cys Ser
980 985 990

Cys Pro Thr Gly Phe Glu Gly Pro Thr Cys Gly Val Asn Thr Asp Asp
995 1000 1005

Cys Val Asp His Ala Cys Ala Asn Gly Gly Val Cys Val Asp Gly
1010 1015 1020

Val Gly Asn Tyr Thr Cys Gln Cys Pro Leu Gln Tyr Glu Gly Lys
1025 1030 1035

Ala Cys Glu Gln Leu Val Asp Leu Cys Ser Pro Asp Leu Asn Pro
1040 1045 1050

Cys Gln His Glu Ala Gln Cys Val Gly Thr Pro Asp Gly Pro Arg
1055 1060 1065

Cys Glu Cys Met Pro Gly Tyr Ala Gly Asp Asn Cys Ser Glu Asn
1070 1075 1080

Gln Asp Asp Cys Arg Asp His Arg Cys Gln Asn Gly Ala Gln Cys
1085 1090 1095

Met Asp Glu Val Asn Ser Tyr Ser Cys Leu Cys Ala Glu Gly Tyr
1100 1105 1110

Ser Gly Gln Leu Cys Glu Ile Pro Pro
1115 1120

<210> 28
<211> 928
<212> PRT
<213> Homo sapiens
<400> 28

Met Ala Leu Thr Pro Gly Trp Gly Ser Ser Ala Gly Pro Val Arg Pro
1 5 10 . 15

Glu Leu Trp Leu Leu Leu Trp Ala Ala Ala Trp Arg Leu Gly Ala Ser
            20                  25                  30

Ala Cys Pro Ala Leu Cys Thr Cys Thr Gly Thr Thr Val Asp Cys His
        35                  40                  45

Gly Thr Gly Leu Gln Ala Ile Pro Lys Asn Ile Pro Arg Asn Thr Glu
    50                  55                  60

Arg Leu Glu Leu Asn Gly Asn Asn Ile Thr Arg Ile His Lys Asn Asp
65                  70                  75                  80

Phe Ala Gly Leu Lys Gln Leu Arg Val Leu Gln Leu Met Glu Asn Gln
                85                  90                  95

Ile Gly Ala Val Glu Arg Gly Ala Phe Asp Asp Met Lys Glu Leu Glu
            100                 105                 110

Arg Leu Arg Leu Asn Arg Asn Gln Leu His Met Leu Pro Glu Leu Leu
        115                 120                 125

Phe Gln Asn Asn Gln Ala Leu Ser Arg Leu Asp Leu Ser Glu Asn Ala
    130                 135                 140

Ile Gln Ala Ile Pro Arg Lys Ala Phe Arg Gly Ala Thr Asp Leu Lys
145                 150                 155                 160

Asn Leu Gln Leu Asp Lys Asn Gln Ile Ser Cys Ile Glu Glu Gly Ala
                165                 170                 175

Phe Arg Ala Leu Arg Gly Leu Glu Val Leu Thr Leu Asn Asn Asn Asn
            180                 185                 190

Ile Thr Thr Ile Pro Val Ser Ser Phe Asn His Met Pro Lys Leu Arg
        195                 200                 205

Thr Phe Arg Leu His Ser Asn His Leu Phe Cys Asp Cys His Leu Ala
    210                 215                 220

Trp Leu Ser Gln Trp Leu Arg Gln Arg Pro Thr Ile Gly Leu Phe Thr
225                 230                 235                 240

Gln Cys Ser Gly Pro Ala Ser Leu Arg Gly Leu Asn Val Ala Glu Val
                245                 250                 255

Gln Lys Ser Glu Phe Ser Cys Ser Gly Gln Gly Glu Ala Gly Arg Val

260                 265                 270

Pro Thr Cys Thr Leu Ser Ser Gly Ser Cys Pro Ala Met Cys Thr Cys
        275                 280                 285

Ser Asn Gly Ile Val Asp Cys Arg Gly Lys Gly Leu Thr Ala Ile Pro
    290                 295                 300

Ala Asn Leu Pro Glu Thr Met Thr Glu Ile Arg Leu Glu Leu Asn Gly
305                 310                 315                 320

Ile Lys Ser Ile Pro Pro Gly Ala Phe Ser Pro Tyr Arg Lys Leu Arg
                325                 330                 335

Arg Ile Asp Leu Ser Asn Asn Gln Ile Ala Glu Ile Ala Pro Asp Ala
            340                 345                 350

Phe Gln Gly Leu Arg Ser Leu Asn Ser Leu Val Leu Tyr Gly Asn Lys
        355                 360                 365

Ile Thr Asp Leu Pro Arg Gly Val Phe Gly Gly Leu Tyr Thr Leu Gln
    370                 375                 380

Leu Leu Leu Leu Asn Ala Asn Lys Ile Asn Cys Ile Arg Pro Asp Ala
385                 390                 395                 400

Phe Gln Asp Leu Gln Asn Leu Ser Leu Leu Ser Leu Tyr Asp Asn Lys
                405                 410                 415

Ile Gln Ser Leu Ala Lys Gly Thr Phe Thr Ser Leu Arg Ala Ile Gln
            420                 425                 430

Thr Leu His Leu Ala Gln Asn Pro Phe Ile Cys Asp Cys Asn Leu Lys
        435                 440                 445

Trp Leu Ala Asp Phe Leu Arg Thr Asn Pro Ile Glu Thr Ser Gly Ala
    450                 455                 460

Arg Cys Ala Ser Pro Arg Arg Leu Ala Asn Lys Arg Ile Gly Gln Ile
465                 470                 475                 480

Lys Ser Lys Lys Phe Arg Cys Ser Ala Lys Glu Gln Tyr Phe Ile Pro
                485                 490                 495

Gly Thr Glu Asp Tyr Gln Leu Asn Ser Glu Cys Asn Ser Asp Val Val
            500                 505                 510

Cys Pro His Lys Cys Arg Cys Glu Ala Asn Val Val Glu Cys Ser Ser
515 520 525

Leu Lys Leu Thr Lys Ile Pro Glu Arg Ile Pro Gln Ser Thr Ala Glu
530 535 540

Leu Arg Leu Asn Asn Asn Glu Ile Ser Ile Leu Glu Ala Thr Gly Met
545 550 555 560

Phe Lys Lys Leu Thr His Leu Lys Lys Ile Asn Leu Ser Asn Asn Lys
565 570 575

Val Ser Glu Ile Glu Asp Gly Ala Phe Glu Gly Ala Ala Ser Val Ser
580 585 590

Glu Leu His Leu Thr Ala Asn Gln Leu Glu Ser Ile Arg Ser Gly Met
595 600 605

Phe Arg Gly Leu Asp Gly Leu Arg Thr Leu Met Leu Arg Asn Asn Arg
610 615 620

Ile Ser Cys Ile His Asn Asp Ser Phe Thr Gly Leu Arg Asn Val Arg
625 630 635 640

Leu Leu Ser Leu Tyr Asp Asn Gln Ile Thr Thr Val Ser Pro Gly Ala
645 650 655

Phe Asp Thr Leu Gln Ser Leu Ser Thr Leu Asn Leu Leu Ala Asn Pro
660 665 670

Phe Asn Cys Asn Cys Gln Leu Ala Trp Leu Gly Gly Trp Leu Arg Lys
675 680 685

Arg Lys Ile Val Thr Gly Asn Pro Arg Cys Gln Asn Pro Asp Phe Leu
690 695 700

Arg Gln Ile Pro Leu Gln Asp Val Ala Phe Pro Asp Phe Arg Cys Glu
705 710 715 720

Glu Gly Gln Glu Glu Gly Gly Cys Leu Pro Arg Pro Gln Cys Pro Gln
725 730 735

Glu Cys Ala Cys Leu Asp Thr Val Val Arg Cys Ser Asn Lys His Leu
740 745 750

Arg Ala Leu Pro Lys Gly Ile Pro Lys Asn Val Thr Glu Leu Tyr Leu
755 760 765

Asp Gly Asn Gln Phe Thr Leu Val Pro Gly Gln Leu Ser Thr Phe Lys
    770                 775                 780

Tyr Leu Gln Leu Val Asp Leu Ser Asn Asn Lys Ile Ser Ser Leu Ser
785                 790                 795                 800

Asn Ser Ser Phe Thr Asn Met Ser Gln Leu Thr Thr Leu Ile Leu Ser
                805                 810                 815

Tyr Asn Ala Leu Gln Cys Ile Leu Pro Leu Ala Phe Gln Gly Leu Arg
            820                 825                 830

Ser Leu Arg Leu Leu Ser Leu His Gly Asn Asp Ile Ser Thr Leu Gln
        835                 840                 845

Glu Gly Ile Phe Ala Asp Val Thr Ser Leu Ser His Leu Ala Ile Gly
    850                 855                 860

Ala Asn Pro Leu Tyr Cys Asp Cys His Leu Arg Trp Leu Ser Ser Trp
865                 870                 875                 880

Val Lys Thr Gly Tyr Lys Glu Pro Gly Ile Ala Arg Cys Ala Gly Pro
                885                 890                 895

Gln Asp Met Glu Gly Lys Leu Leu Leu Thr Thr Pro Ala Lys Lys Phe
            900                 905                 910

Glu Cys Gln Gly Pro Pro Thr Leu Ala Val Gln Ala Lys Cys Asp Leu
        915                 920                 925